# EUROPEAN PATENT SPECIFICATION

(11) **EP 3 917 875 B1**
(45) Date of publication and mention of the grant of the patent: **03.06.2026**
(21) Application number: 20701501.7
(22) Date of filing: 02.01.2020
(51) Int. Cl.: C01B 3/38, C07C 27/06, C07C 41/01, C07C 41/09, C07C 51/12, C07C 53/08, C10K 3/02

(54) **A PROCESS FOR PRODUCING HYDROGEN-LEAN SYNGAS FOR ACETIC ACID SYNTHESIS AND DIMETHYL ETHER SYNTHESIS**
VERFAHREN ZUR HERSTELLUNG VON WASSERSTOFFARMEM SYNGAS FÜR DIE ESSIGSÄURE- UND DIMETHYLETHERSYNTHESE
PROCÉDÉ DE PRODUCTION DE GAZ DE SYNTHÈSE PAUVRE EN HYDROGÈNE POUR LA SYNTHÈSE D'ACIDE ACÉTIQUE ET LA SYNTHÈSE D'ÉTHER DIMÉTHYLIQUE

(30) Priority: 28.01.2019 US 201962797591 P
(43) Date of publication of application: 08.12.2021
(73) Proprietor: ENI S.p.A., 00144 Roma (IT)
(72) Inventor: RAJAGOPALAN, Vijayanand, Bengaluru 562125 (IN); PANT, Atul, Bengaluru 562125 (IN); NARAYANASWAMY, Ravichander, Bengaluru 562125 (IN)
(74) Representative: Bottero, Carlo
(86) International application number: PCT/IB2020/050012
(87) International publication number: WO 2020/157585

(56) References cited:
- EP-A1- 0 984 826
- WO-A1-2018/234971
- US-A- 5 286 900
- US-A1- 2004 013 605
- US-A1- 2006 128 818
- US-B1- 6 521 783

## Description

### TECHNICAL FIELD

The present disclosure relates to methods for producing acetic acid and dimethyl ether (DME); more specifically, the present disclosure elates to systems and methods of producing acetic acid and DME from a hydrogen-lean synthesis gas, wherein the hydrogen-lean synthesis gas is produced via catalytic partial oxidation (CPO); still more specifically, the present disclosure prelates to methods for producing acetic acid and DME via CPO of a CPO reactant mixture comprising hydrocarbons and oxygen, wherein the hydrocarbons comprise greater than or equal to about 3 mole percent (mol%) of higher hydrocarbons (e.g., alkanes comprising 2 or more carbons, C₂₊).

### BACKGROUND

Synthesis gas (syngas) is a mixture comprising carbon monoxide (CO) and hydrogen (H₂), as well as small amounts of carbon dioxide (CO₂), water (H₂O), and unreacted methane (CH₄). Syngas is generally used as an intermediate in a variety of synthesis processes, including, without limitation, dimethyl ether (DME), alcohols, such as methanol, ethanol, oxoalcohols (e.g., n-butanol etc.), ethylene glycol, aldehydes, and the like. Syngas is produced conventionally by steam reforming of natural gas (steam methane reforming or SMR), although other hydrocarbon sources can be used for syngas production, such as refinery off-gases, naphtha feedstocks, heavy hydrocarbons, coal, biomass, etc. SMR is an endothermic process and requires significant energy input to drive the reaction forward. Conventional endothermic technologies such as SMR produce syngas with a H₂ content greater than the required for a variety of downstream chemical syntheses. In an autothermal reforming (ATR) process, a portion of the natural gas is burned as fuel to drive the conversion of natural gas to syngas resulting in relatively low hydrogen and high CO₂ concentrations. Conventional combined reforming (CR) technology pairs SMR with autothermal reforming (ATR) to reduce the amount of H₂ present in syngas. ATR produces a syngas with a lower hydrogen content. CR syngas generally has a H₂ content greater than needed for many downstream synthesis processes. Furthermore, SMR is a highly endothermic process, and the endothermicity of the SMR technology requires burning fuel to drive the syngas synthesis. Consequently, SMR technology reduces the energy efficiency of downstream chemical synthesis process.

Syngas can also be produced (non-commercially) by catalytic partial oxidation (CPO or CPOx) of natural gas. CPO processes employ partial oxidation of hydrocarbon feeds to syngas comprising CO and H₂. The CPO process is exothermic, thus eliminating the need for external heat supply. Conventional partial oxidation processes do not produce H₂-lean synthesis suitable for use in downstream syntheses requiring molar ratios of hydrogen to carbon monoxide less than about 1.6. Thus, there is an ongoing need for the development of systems and methods of producing acetic acid and DME from H₂-lean syngas produced via CPO.

US6521783B1 relates to a processes for the conversion of a feedstock stream comprising carbon monoxide and hydrogen to a product stream comprising at least one of an ester, acid, acid anhydride and mixtures thereof. This invention also relates to processes for converting an alcohol and/or ether feedstock to oxygenated products, e.g., esters, acids, acid anhydrides and mixtures thereof. The processes and catalysts are especially suitable for the production of acetic acid and methyl acetate from a synthesis gas feedstock or from an alcohol and/or ether feedstock.

### BRIEF DESCRIPTION OF THE DRAWINGS

For a detailed description of the preferred embodiments of the disclosed methods, reference will now be made to the accompanying drawing in which:

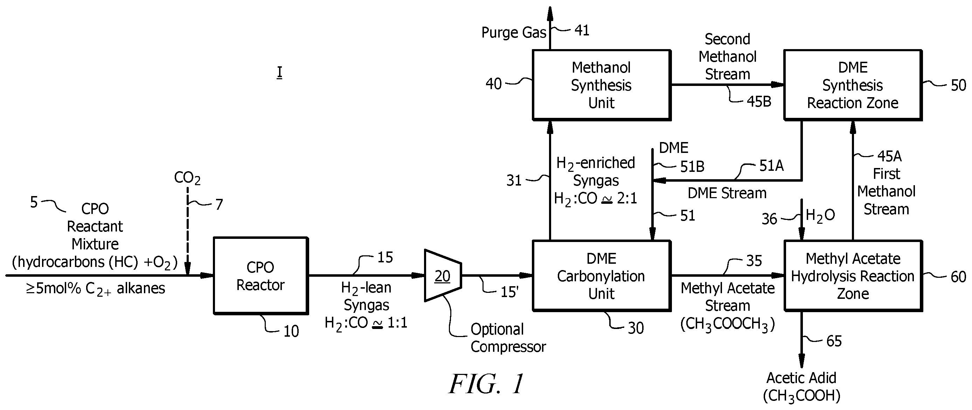

Figure 1 is a schematic of a system I for the production of acetic acid from a hydrogen-lean synthesis gas produced via catalytic partial oxidation (CPO or CPOx), according to embodiments of this disclosure;

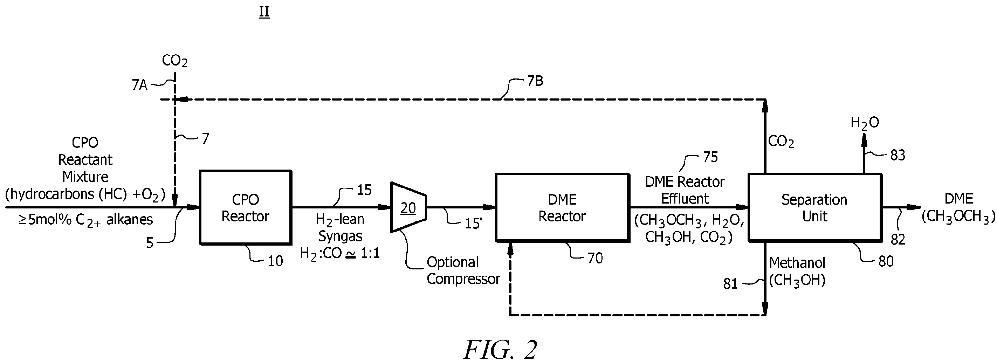

Figure 2 is a schematic of a system II for the production of DME from a hydrogen-lean synthesis gas produced via CPO, according to embodiments of this disclosure;

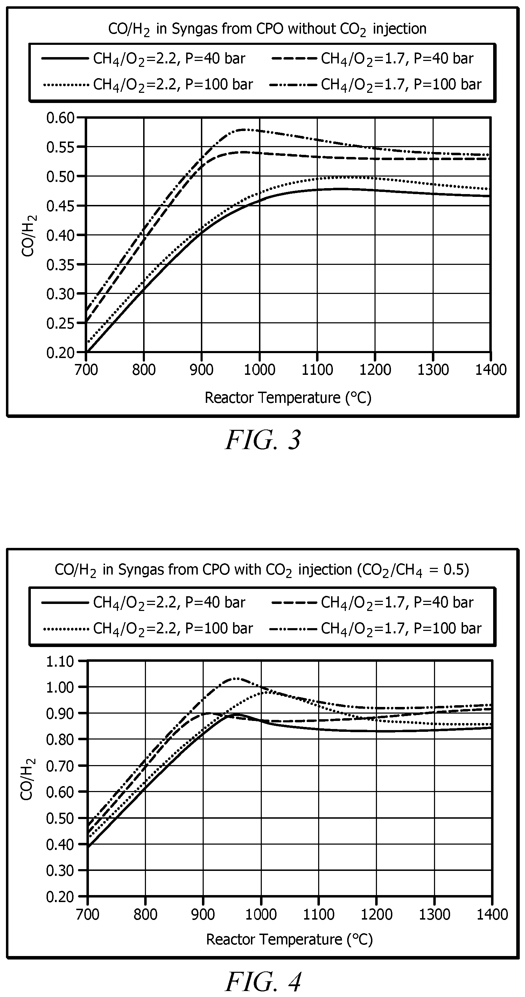

Figure 3 is a plot of the molar ratio of carbon monoxide to hydrogen (CO/H₂) in syngas from CPO as a function of reactor temperature without CO₂ injection in the reactant feed;
Figure 4 is a plot of the molar ratio of CO/H₂ in syngas from CPO as a function of reactor temperature with CO₂ injection for a reactant feed comprising a molar ratio of carbon dioxide to methane (CO₂/CH₄) of 0.5;

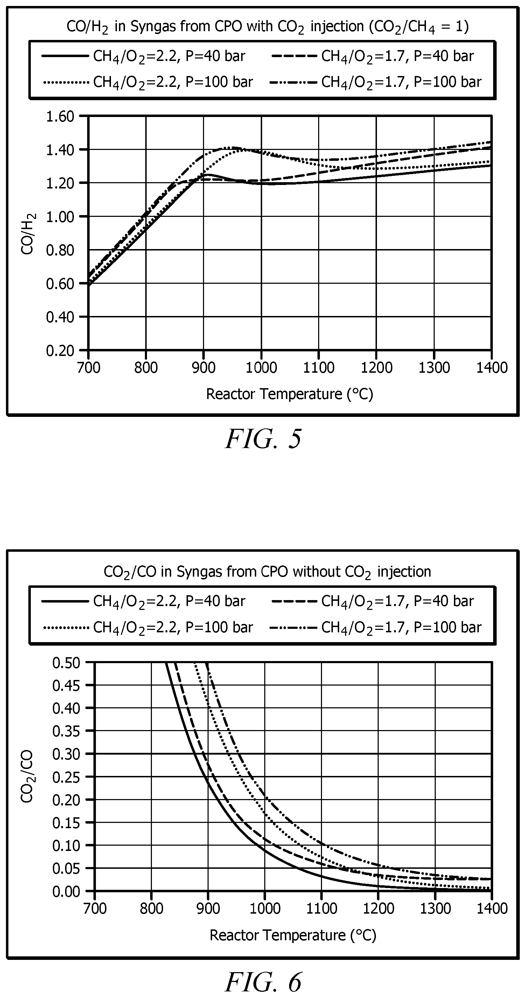

Figure 5 is a plot of the molar ratio of CO/H₂ in syngas from CPO as a function of reactor temperature with CO₂ injection for a reactant feed comprising a molar ratio of carbon dioxide to methane (CO₂/CH₄) of 1;
Figure 6 is a plot of molar ratio of CO₂/CO in syngas from CPO as a function of reactor temperature without CO₂ injection for reactant feeds comprising a molar ratio of CH₄/O₂ of 2.2, 1.7 and pressures of 40, 100 bar;

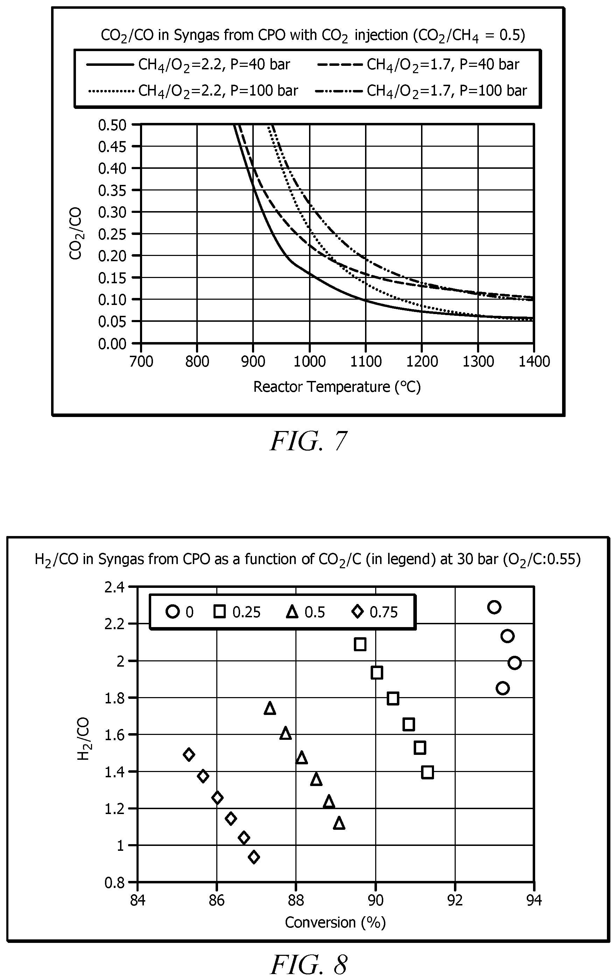

Figure 7 is a plot of the molar ratio of CO₂/CO in syngas from CPO as a function of reactor temperature with CO₂ injection for a reactant feed comprising a molar ratio of carbon dioxide to methane (CO₂/CH₄) of 0.5;
Figure 8 is a plot showing the molar ratio of carbon monoxide to hydrogen (H₂/CO) in syngas from CPO as a function of the conversion (%) and the molar ratio of carbon dioxide to carbon (CO₂/C) in the reactant feed (in legend) at a pressure of 30 bar and an oxygen to carbon molar ratio (O₂/C) of 0.55;

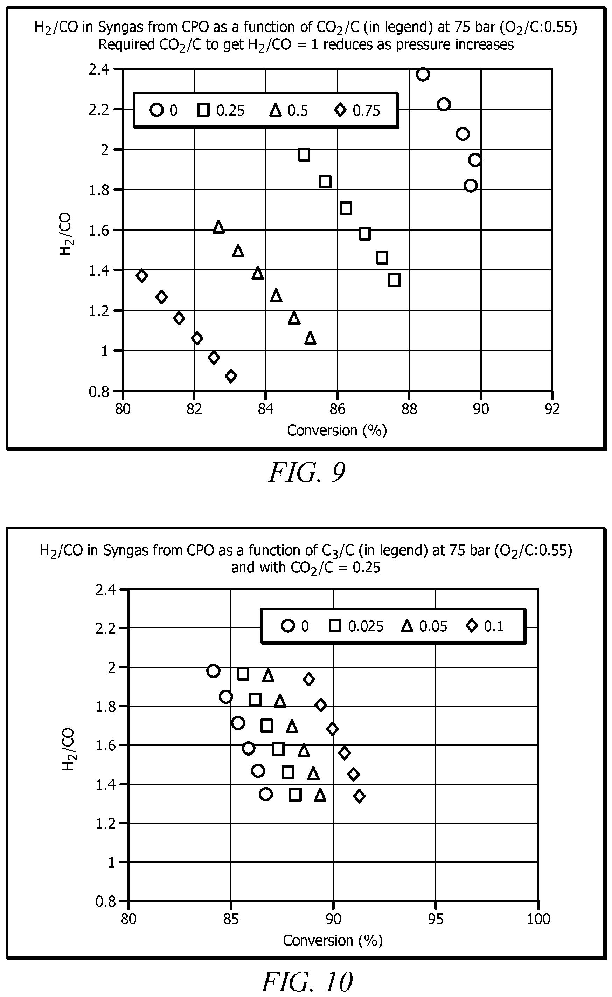

Figure 9 is a plot showing the molar ratio of carbon monoxide to hydrogen (H₂/CO) in syngas from CPO as a function of the conversion (%) and the molar ratio of carbon dioxide to carbon (CO₂/C) in the reactant feed (in legend) at a pressure of 75 bar and an oxygen to carbon molar ratio (O₂/C) of 0.55;
Figure 10 is a plot showing the molar ratio of carbon monoxide to hydrogen (H₂/CO) in syngas from CPO as a function of the conversion (%) and the molar ratio of hydrocarbons having three carbons (C₃) to carbon (C₃/C) in the reactant feed (in legend) at a pressure of 75 bar, an oxygen to carbon molar ratio (O₂/C) of 0.55, and a carbon dioxide to carbon (CO₂/C) molar ratio of 0.25;

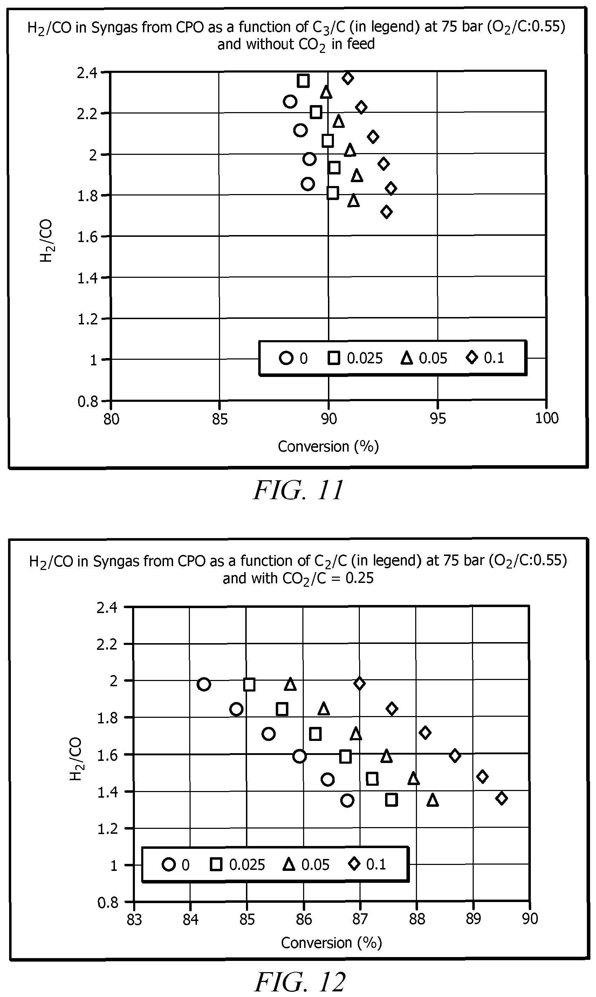

Figure 11 is a plot showing the molar ratio of carbon monoxide to hydrogen (H₂/CO) in syngas from CPO as a function of the conversion (%) and the molar ratio of hydrocarbons having three carbons (C₃) to carbon (C₃/C) in the reactant feed (in legend) at a pressure of 75 bar, an oxygen to carbon molar ratio (O₂/C) of 0.55, and without CO₂ in the reactant feed;
Figure 12 is a plot showing the molar ratio of carbon monoxide to hydrogen (H₂/CO) in syngas from CPO as a function of the conversion (%) and the molar ratio of hydrocarbons having two carbons (C₂) to carbon (C₂/C) in the reactant feed (in legend) at a pressure of 75 bar, an oxygen to carbon molar ratio (O₂/C) of 0.55, and a carbon dioxide to carbon (CO₂/C) molar ratio of 0.25;

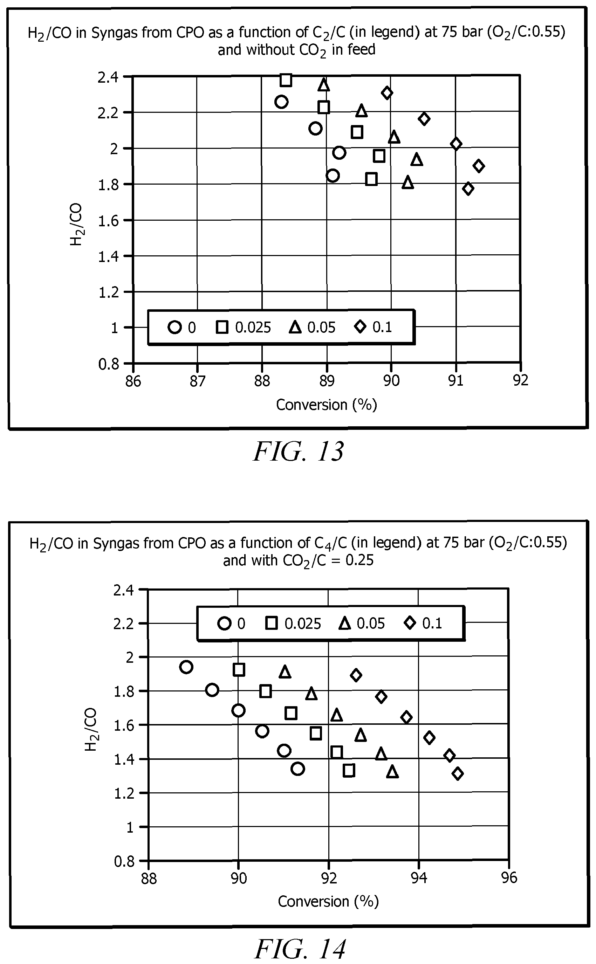

Figure 13 is a plot showing the molar ratio of carbon monoxide to hydrogen (H₂/CO) in syngas from CPO as a function of the conversion (%) and the molar ratio of hydrocarbons having two carbons (C₂) to carbon (C₂/C) in reactant feed (in legend) at a pressure of 75 bar, O₂/C molar ratio of 0.55, and without CO₂ in reactant feed;
Figure 14 is a plot showing the molar ratio of carbon monoxide to hydrogen (H₂/CO) in syngas from CPO as a function of the conversion (%) and the molar ratio of hydrocarbons having four carbons (C₄) to carbon (C₄/C) in the reactant feed (in legend) at a pressure of 75 bar, an oxygen to carbon molar ratio (O₂/C) of 0.55, and a carbon dioxide to carbon (CO₂/C) molar ratio of 0.25; and

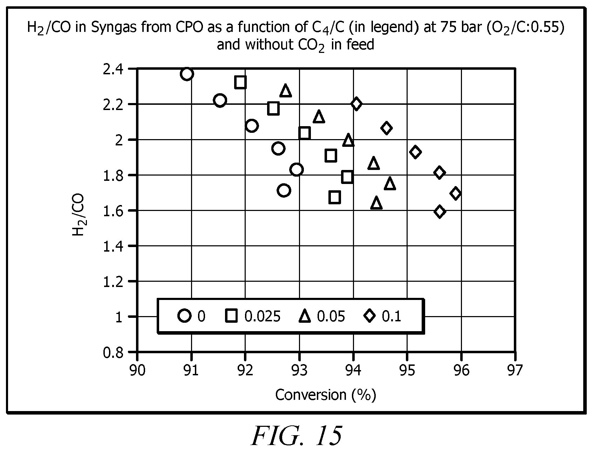

Figure 15 is a plot showing the molar ratio of carbon monoxide to hydrogen (H₂/CO) in syngas from CPO as a function of the conversion (^{%}) and the molar ratio of hydrocarbons having four carbons (C₄) to carbon (C₄/C) in reactant feed (in legend) at a pressure of 75 bar, O₂/C molar ratio of 0.55, and without CO₂ in reactant feed.

### DETAILED DESCRIPTION

The synthesis gas feeds for a variety of chemical synthesis processes require H₂-lean synthesis gas having a molar ratio of H₂ to carbon monoxide (H₂/CO) of about 1:1. When the synthesis gas is produced from conventional processes that provide synthesis gas having a higher molar ratio (e.g., about 2: 1), synthesis gas has to be pretreated, for example via a H₂ removal unit (e.g., a pressure swing adsorption PSA unit), to reduce the H₂/CO molar ration of synthesis gas. Conventional partial oxidation (POx) processes do not provide syngas having a H₂/CO molar ratio of about 1 : 1. Using an intermediate H₂ removal (e.g., PSA) step increases energy and capital cost requirements.

According to this disclosure, hydrogen-lean syngas (e.g., syngas having a molar ratio of C in the range of from 0.8 to 1.6) can be produced via a catalytic partial oxidation (CPO) process. Via embodiments of the herein disclosed method, a CPO process can be tailored to provide a hydrogen-lean syngas having a desired composition (e.g., a reduced H₂/CO molar ratio relative to that of a syngas produced by a conventional POX process). Accordingly, the herein disclosed systems and methods can reduce the size of or eliminate hydrogen removal apparatus, thus reducing the number of unit operations, and thus can, in embodiments, also reduce energy requirements for the process.

In embodiments, CPO is utilized to produce a hydrogen-lean synthesis gas by utilizing a CPO reactant feed that comprises higher hydrocarbons and/or carbon dioxide (CO₂). The use of reactant feeds comprising higher hydrocarbons can allow for a reduction of the amount of the CO₂ required to reach an H₂/CO molar ratio of about 1 and at the same time enable for production of hydrogen-lean syngas having the desired H₂/CO molar ratio of about 1 at a higher conversion of hydrocarbon to syngas.

Other than in the operating examples or where otherwise indicated, all numbe1S or expressions referring to quantities of ingredients, reaction conditions, and the like, used in the specification and claims are to be understood as modified in all instances by the term "about." Various numerical ranges are disclosed herein. Because these ranges are continuous, they include every value between the minimum and maximum values. The endpoints of all ranges reciting the same characteristic or component are independently combinable and inclusive of the recited endpoint.

Unless expressly indicated otherwise, the various numerical ranges specified in this application are approximations. The endpoints of all ranges directed to the same component or property are inclusive of the endpoint and independently combinable. The term "from more than 0 to an amount" means that the named component is present in some amount more than 0, and up to and including the higher named amount.

The terms "a," "an," and 'the" do not denote a limitation of quantity, but rather denote the presence of at least one of the referenced item. As used herein the singular forms "a," "an," and 'the" include plural referents.

As used herein, "combinations thereof' is inclusive of one or more of the recited elements, optionally together with a like element not recited, e.g., inclusive of a combination of one or more of the named components, optionally with one or more other components not specifically named that have essentially the same function. As used herein, the term "combination" is inclusive of blends, mixtures, alloys, reaction products, and the like.

Reference throughout the specification to "an embodiment," "another embodiment," "other embodiments, 'some embodiments," and so forth, means that a particular element (e.g., feature, structure, property,
and/or characteristic) described in connection with the embodiment is included in at least an embodiment described herein, and may or may not be present in other embodiments. In addition, it is to be understood that the described element(s) can be combined in any suitable manner in the various embodiments.

As used herein, the terms "inhibiting" or "reducing" or "preventing" or "avoiding" or any variation of these terms, include any measurable decrease or complete inhibition to achieve a desired result. As used herein, the term "effective," means adequate to accomplish a desired, expected, or intended result. As used herein, the terms "comprising" (and any form of comprising, such as "comprise" and "comprises"), "having" (and any form of having, such as "have" and "has"), "including" (and any form of including, such as "include" and "includes") or "containing" (and any form of containing, such as "contain" and "contains") are inclusive or open-ended and do not exclude additional, unrecited elements or method steps.

Unless defined otherwise, technical and scientific terms used herein have the same meaning as is commonly understood by one of skill in the alt. Compounds are described herein using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through the carbon of the carbonyl group. As used herein, the terms "Cx hydrocarbons" and "Cxs" are interchangeable and refer to any hydrocarbon having x number of carbon atoms (C). For example, the terms "C₄ hydrocarbons" and "C₄ₛ" both refer to any hydrocarbons having exactly 4 carbon atoms, such as Il-butane, iso-butane, cyclobutane, 1-butene, 2-butene, isobutylene, butadiene, and the like, or combinations thereof. As used herein, the term "Cₓ₊ hydrocarbons" refers to any hydrocarbon having greater than or equal to x carbon atoms (C). For example, the term "C₂₊ hydrocarbons" refers to any hydrocarbons having 2 or more carbon atoms, such as ethane, ethylene, C₃s, C₄s, C₅s, etc.

Referring to Figure 1, a chemical production system I is disclosed. The chemical production system I generally comprises a catalytic partial oxidation (CPO or CPOx) reactor 10 and a downstream synthesis apparatus comprising acetic acid synthesis apparatus (Figure 1) or DME synthesis apparatus (Figure 2). As will be appreciated by one of skill in the art, and with the help of this disclosure, methanol production system components shown in Figure 1 and Figure 2 can be in fluid communication with each other (as represented by the connecting lines indicating a direction of fluid flow) through any suitable conduits (e.g., pipes, streams, etc.).

In embodiments, a process for producing DME or acetic acid as disclosed herein can comprise a step of reacting, via a catalytic partial oxidation (CPO) reaction, a CPO reactant mixture 5 in a CPO reactor 10 to produce a hydrogen-lean syngas 15; wherein the CPO reactant mixture 5 comprises hydrocarbons and oxygen (O₂) and carbon dioxide (CO₂); wherein the hydrocarbons comprise greater than or equal to 3 mol% C₂₊ alkanes; wherein the CPO reactor 10 comprises a CPO catalyst; wherein the hydrogen-lean syngas 15 comprises hydrogen, carbon monoxide (CO), CO₂, and unreacted hydrocarbons; and wherein the hydrogen-lean syngas 15 is characterized by a hydrogen to carbon monoxide (H₂/CO) molar ratio of from 0.8 to 1,6.

Generally, the CPO reaction is based on partial combustion of fuels, such as various hydrocarbons, and in the case of methane, CPO can be represented by equation (1):

CH₄ + 1/2 O₂ → CO + 2 H₂ (1)

Without wishing to be limited by theory, side reactions can take place along with the CPO reaction depicted in equation (1); and such side reactions can produce CO₂ and water (H₂O), for example via hydrocarbon combustion, which is an exothermic reaction. As will be appreciated by one of skill in the art, and with the help of this disclosure, and without wishing to be limited by theory, the CPO reaction as represented by equation (1) can yield a syngas with a H₂/CO molar ratio having the theoretical stoichiometric limit of 2.0. Without wishing to be limited by theory, the theoretical stoichiometric limit of 2.0 for the H₂/CO molar ratio means that the CPO reaction as represented by equation (1) yields 2 moles of H₂ for every 1 mole of CO, i.e., H₂/CO molar ratio of (2 moles H₂/1 mole CO) = 2. As will be appreciated by one of skill in the art, and with the help of this disclosure, the theoretical stoichiometric limit of 2.0 for the H₂/CO molar ratio in a CPO reaction cannot be achieved practically because reactants (e.g., hydrocarbons, O₂) as well as products (e.g., H₂, CO) undergo side reactions at the conditions used for the CPO reaction. As will be appreciated by one of skill in the art, and with the help of this disclosure, and without wishing to be limited by theory, in the presence of O₂, CO and H₂ can be oxidized to CO₂ and H₂O, respectively. The relative amounts (e.g., composition) of CO, H₂, CO₂ and H₂O can be further altered by the equilibrium of the water-gas shift (WGS) reaction, which will be discussed in more detail later herein. The side reactions that can take place in the CPO reactor 10 can have a direct impact on the composition of the hydrogen-lean syngas 15 which, according to this disclosure comprises the hydrogen-lean syngas. In the absence of any side reaction (theoretically), the CPO reaction as represented by equation (1) results in a syngas with an H₂/CO molar ratio of 2.0. However, the presence of side reactions can (practically) reduce H₂ (and increase CO₂), thereby resulting in a syngas with a reduced H₂/CO molar ratio.

Further, without wishing to be limited by theory, the CPO reaction as depicted in equation (1) is an exothermic heterogeneous catalytic reaction (i.e., a mildly exothermic reaction) and it occurs in a single reactor unit, such as the CPO reactor 10 (as opposed to more than one reactor unit as is the case in conventional processes for syngas production, such as steam methane reforming (SMR) - autothermal reforming (ATR) combinations). While it is possible to conduct partial oxidation of hydrocarbons as a homogeneous reaction, in the absence of a catalyst, homogeneous partial oxidation of hydrocarbons process entails excessive temperatures, long residence times, as well as excessive coke formation, which strongly reduce the controllability of the partial oxidation reaction, and may not produce syngas of the desired quality in a single reactor unit. Furthermore, without wishing to be limited by theory, the CPO reaction is fairly resistant to chemical poisoning, and as such it allows for the use of a wide variety of hydrocarbon feedstocks, including some sulfur containing hydrocarbon feedstocks; which, in some cases, can enhance catalyst life-time and productivity. By contrast, conventional ATR processes have more restrictive feed requirements, for example in terms of content of impurities in the feed (e.g., feed to ATR is desulfurized), as well as hydrocarbon composition (e.g., ATR primarily uses a CH₄-rich feed).

In embodiments, the hydrocarbons suitable for use in a CPO reaction as disclosed herein can include methane, natural gas, natural gas liquids, liquefied petroleum gas (LPG), associated gas, well head gas, enriched gas, paraffins, shale gas, shale liquids, fluid catalytic cracking (FCC) off gas, refinery process gases, refinery off gases, stack gases, fuel gas from a fuel gas header, or combinations thereof. In embodiments, an amount of CO₂ and/or CO in the reactant mixture 5 can be increased by diluting a feed with gases (e.g., stack gases) containing CO₂ and/or CO. Such gases containing CO₂ and/or CO include, without limitation, stack gases, reducing gases, off gases rich in CO, such as used in the metal industry, crackers, and the like. For example, dedicated coking reactors can be utilized which, when injected with steam supply, air, and CO₂ deliver a continuous CO stream to CPO reactor 10.

In embodiments, reactant mixture 5 comprises fuel gases from a steam cracker and CPO reactor 10 is operated at a high CH₄O₂ molar ratio by providing an autothermal mode of operation. In embodiments, a hydrogen content of the reactant mixture 5 can be adjusted to maintain an appropriate adiabatic rise.

The hydrocarbons can include any suitable hydrocarbons source, and can contain C₁-C₆ hydrocarbons, as well some heavier hydrocarbons. In embodiments, the CPO reactant mixture 5 can comprise natural gas. Generally, natural gas is composed primarily of methane, but can also contain ethane, propane and heavier hydrocarbons (e.g., iso-butane, n-butane, iso-pentane, n-pentane, hexanes, etc.), as well as very small quantities of nitrogen (N₂), O₂, CO₂, sulfur compounds, and/or water. The natural gas can be provided from a variety of sources including, but not limited to, gas fields, oil fields, coal fields, fracking of shale fields, biomass, landfill gas, and the like, or combinations thereof. In some aspects, the CPO reactant mixture 5 can comprise primarily CH₄ and O₂.

The natural gas can comprise any suitable amount of methane. In some embodiments, the natural gas can comprise biogas. For example, the natural gas can comprise from about 45 mol% to about 80 mol% methane, from about 20 mol% to about 55 mol% CO₂, and less than about 15 mol% N₂.

In embodiments, natural gas can comprise CH4 in an amount of greater than or equal to about 45 mol%, about 50 mol%, about 55 mol%, about 60 mol%, about 65 mol%, about 70 mol%, about 75 mol%, about 80 mol%, about 82 mol%, about 84 mol%, about 86 mol%, about 88 mol%, about 90 mol%, about 91 mol%, about 92 mol%, about 93 mol%, about 94 mol%, about 95 mol%, about 96 mol%, or about 97 mol%.

According to this disclosure, the hydrocarbons in the reactant feed 5 comprise greater than or equal to about 3, 4, 5, 6, 7, 8, 9, or 10 mol% of heavier hydrocarbons comprising hydrocarbons having two or more carbons (e.g., C₂₊ hydrocarbons). In embodiments, the hydrocarbons in the reactant feed 5 comprise greater than or equal to about 3, 4, 5, 6, 7, 8, 9, or 10 mol% of C₂₊ alkanes. In embodiments, the hydrocarbons in reactant feed 5 comprise ethane in an amount of greater than or equal to about 4, 5, 6, 7, 8, 9, 10, 11, 12, 13, 14, or 15 mol%. In embodiments, the hydrocarbons in the reactant feed 5 comprise propane in an amount of greater than or equal to about 4, 5, 6, 7, 8, 9, 10, 11, 12, 13, 14, or 15 mol%. In embodiments, hydrocarbons comprise butanes in an amount of greater than or equal to about 3, 4, 5, 6, 7, or 8 mol%. In embodiments, the hydrocarbons in reactant feed 5 comprise ethane in an amount of greater than or equal to about 4, 5, 6, 7, 8, 9, 10, 1 1, 12, 13, 14, or 15 mol%, propane in an amount of greater than or equal to about 4, 5, 6, 7, 8, 9, 10, 1 1, 12, 13, 14, or 15 mol%, butanes in an amount of greater than or equal to about 3, 4, 5, 6, 7, or 8 mol%, or a combination thereof.

In embodiments, the CPO reactant mixture 5 is characterized by a CO₂ to carbon (CO₂/C) and/or a CO₂/CH₄ molar ratio in the CPO reactant mixture 5 of greater than or equal to 0.5:1, 0.25:1, or 0:1, wherein the CO₂/C molar ratio refers to the total moles of CO₂ in the reactant mixture divided by the total moles of carbon (C) in the hydrocarbons in the reactant mixture 5. In embodiments, the CPO reactant mixture 5 is characterized by a CO₂ to carbon (CO₂/C) molar ratio in the CPO reactant mixture 5 of less than or equal to 10:1, 5:1, or 2:1. All or a portion of the CO₂ in reactant feed 5 can be introduced into the reactant mixture 5 via CO₂ stream 7, in embodiments. In embodiments, CPO reactor 10 is operated in autothermal mode with CO₂ injection or addition via 7.

In embodiments, the amount of CO₂ in the CPO reactant mixture 5 is lower than the amount of CO₂ in a CPO reactant mixture in an otherwise similar process that produces a hydrogen-lean syngas from a reactant feed comprising a lower quantity of C₂₊ alkanes hydrocarbons (e.g., wherein the hydrocarbons in the reactant feed 5 comprise less than about 3 mol% C₂₊ alkanes). In embodiments, a portion of the CO₂ in the CPO reactor 10 undergoes a reverse water-gas shift (r-WGS) reaction within CPO reactor 10 (and/or in a separate r-WGS reactor 20 downstream of CPO reactor 10), thereby decreasing the amount of hydrogen in the hydrogen-lean syngas.

In some embodiments, the hydrocarbons suitable for use in a CPO reaction as disclosed herein can comprise C₁-C₆ hydrocarbons (e.g., including C₂, C₃, and/or C₄ as described above), N₂ (e.g., from about 0.1 mol% to about 15 mol%, alternatively from about 0.5 mol% to about 11 mol%, alternatively from about 1 mol% to about 7.5 mol%, or alternatively from about 1.3 mol% to about 5.5 mol%), and CO₂ (e.g., from about 0.1 mol% to about 2 mol%, alternatively from about 0.2 mol% to about 1 mol%, or alternatively from about 0.3 mol% to about 0.6 mol%). For example, the hydrocarbons suitable for use in a CPO reaction as disclosed herein can comprise C₁ hydrocarbon (about 89 mol% to about 92 mol%); C₂ hydrocarbons (greater than or equal to about 4, 5, 6, 7, 8, 9, 10, 11, 12, 13, 14, or 15 mol%); C₃ hydrocarbons (greater than or equal to about 4, 5, 6, 7, 8, 9, 10, 11, 12, 13, 14, or 15 mol%); C₄ hydrocarbons (greater than or equal to about 3, 4, 5, 6, 7, or 8 mol%); C₅ hydrocarbons (about 0.06 mol%); and C₆ hydrocarbons (about 0.02 mol%); and optionally N₂ (about 0.1 mol% to about 15 mol%), CO₂ (about 0.1 mol% to about 2 mol%), or both N₂ (about 0.1 mol% to about 15 mol%) and CO₂ (about 0.1 mol% to about 2 mol%).

The O₂ used in the CPO reactant mixture 5 can comprise 100% O₂ (substantially pure O₂), O₂ gas (which may be obtained via a membrane separation process), technical O₂ (which may contain some air), air, O₂ enriched air, O₂-containing gaseous compounds (e.g., NO), O₂-containing mixtures (e.g., O₂/CO₂, O₂/H₂O, O₂/H₂O₂/H₂O), oxy radical generators (e.g., CH₃OH, CH₂O), hydroxyl radical generators, and the like, or combinations thereof.

In embodiments, the CPO reactant mixture 5 can be characterized by a carbon to oxygen (C/O) or CH₄/O₂ molar ratio of less than about 3:1, about 2.6:1, about 2.4:1, about 2.2:1, about 2:1, or about 1.9:1, alternatively greater than or equal to about 0.1:1, about 0.2:1, about 0.3:1, about 0.4:1, or about 0.5:1, alternatively from about 0.5:1 to about 0.6:1, alternatively from about 0.55:1 to about 0.6:1, alternatively from about 0.5:1 to about 3:1, alternatively from about 0.7:1 to about 2.5:1, alternatively from about 0.9:1 to about 2.2:1, alternatively from about 1:1 to about 2:1, alternatively from about 1.5:1 to about 1.9:1, alternatively from about 2:1 to about 3:1, alternatively from about 2.2:1 to about 3:1, alternatively from about 2.4:1 to about 3:1, or alternatively from about 2.6:1 to about 3:1, wherein the C/O molar ratio refers to the total moles of carbon (C) of hydrocarbons in the reactant mixture 5 divided by the total moles of O₂ in the reactant mixture 5.

As the CPO reactant mixture 5 of this disclosure contains other carbon sources besides CH₄, such as ethane (C₂H₆), propane (C₃H₈), butanes (C₄H₁₀), etc., the C/O molar ratio accounts for the moles of carbon in each compound (e.g., 2 moles of C in 1 mole of C₂H₆, 3 moles of C in 1 mole of C₃H₈, 4 moles of C in 1 mole of C₄H₁₀, etc.). As will be appreciated by one of skill in the art, and with the help of this disclosure, the C/O molar ratio in CPO reactant mixture 5 can be adjusted along with other reactor process parameters (e.g., temperature, pressure, flow velocity, etc.) to provide for a hydrogen-lean syngas as described herein. The C/O molar ratio in CPO reactant mixture 5 can be adjusted to provide for a decreased amount of unconverted hydrocarbons in the syngas. The C/O molar ratio in CPO reactant mixture 5 can be adjusted based on the CPO effluent temperature in order to decrease (e.g., minimize) the unconverted hydrocarbons content of the hydrogen-lean syngas 15 comprising the hydrogen-lean syngas.

In embodiments, a CPO reactor suitable for use in the present disclosure (e.g., CPO reactor 10) can comprise a tubular reactor, a continuous flow reactor, a fixed bed reactor, a fluidized bed reactor, a moving bed reactor, a circulating fluidized bed reactor (e.g., a riser type reactor), a bubbling bed reactor, an ebullated bed reactor, a rotary kiln reactor, and the like, or combinations thereof. In some embodiments, the CPO reactor can comprise a circulating fluidized bed reactor, such as a riser type reactor.

In some embodiments, the CPO reactor 10 can be characterized by at least one CPO operational parameter selected from the group consisting of a CPO reactor temperature (e.g., CPO catalyst bed temperature); CPO feed temperature (e.g., temperature of CPO reactant mixture 5; target temperature of hydrogen-lean syngas 15; a CPO pressure (e.g., pressure of CPO reactor 10); a CPO contact time (e.g., CPO reactor 10 contact time); a C/O molar ratio in the CPO reactant mixture 5; a steam to carbon (S/C) molar ratio in the CPO reactant mixture 5, wherein the S/C molar ratio refers to the total moles of water (H₂O) in the reactant mixture 5 divided by the total moles of carbon (C) of hydrocarbons in the reactant mixture 5; and combinations thereof. For purposes of the disclosure herein, the CPO effluent temperature is the temperature of the syngas (e.g., hydrogen-lean syngas 15) measured at the point where the syngas exits the CPO reactor (e.g., CPO reactor 10), e.g., a temperature of the syngas measured at a CPO reactor outlet, a temperature of the syngas reactor effluent, a temperature of the exit syngas effluent. For purposes of the disclosure herein, the CPO effluent temperature (e.g., target CPO effluent temperature) is considered an operational parameter. As will be appreciated by one of skill in the art, and with the help of this disclosure, the choice of operational parameters for the CPO reactor such as CPO feed temperature; CPO pressure; CPO contact time; C/O molar ratio in the CPO reactant mixture; S/C molar ratio in the CPO reactant mixture; etc. determines the temperature of the hydrogen-lean syngas 15, as well as the composition of the syngas effluent (e.g., hydrogen-lean syngas 15). Further, and as will be appreciated by one of skill in the art, and with the help of this disclosure, monitoring the CPO effluent temperature can provide feedback for changing other operational parameters (e.g., CPO feed temperature; CPO pressure; CPO contact time; C/O molar ratio in the CPO reactant mixture; S/C molar ratio in the CPO reactant mixture; etc.) as necessary for the CPO effluent temperature to match the target CPO effluent temperature. Furthermore, and as will be appreciated by one of skill in the art, and with the help of this disclosure, the target CPO effluent temperature is the desired CPO effluent temperature, and the CPO effluent temperature (e.g., measured CPO effluent temperature, actual CPO effluent temperature) may or may not coincide with the target CPO effluent temperature. In embodiments where the CPO effluent temperature is different from the target CPO effluent temperature, one or more CPO operational parameters (e.g., CPO feed temperature; CPO pressure; CPO contact time; C/O molar ratio in the CPO reactant mixture; S/C molar ratio in the CPO reactant mixture; etc.) can be adjusted (e.g., modified) in order for the CPO effluent temperature to match (e.g., be the same with, coincide with) the target CPO effluent temperature. The CPO reactor 10 can be operated under any suitable operational parameters as described herein that can provide for a hydrogen-lean syngas 15 with a H₂/CO molar ratio in a range of from about 0.8 to 1.6, from about 0.8 to 1.5, from about 0.8 to 1.4, from about 0.8 to 1.3, from about 0.8 to about 1.2, from about 0.9 to about 1.1, or equal to about 1. In embodiments, the hydrogen-lean syngas 15 has a H₂/CO molar ratio in a range of from about 0.7, 0.8, or 0.9 to about 1.1, 1.2, 1.3, 1.4, 1.5, or 1.6.

The CPO reactor 10 can be characterized by a CPO reactant feed temperature of from about 25 °C to about 600 °C, alternatively from about 25 °C to about 500 °C, alternatively from about 25 °C to about 400 °C, alternatively from about 50 °C to about 400 °C, alternatively from about 100 °C to about 400 °C, or alternatively from about 100 °C to about 500 °C. In embodiments, the CPO reactor 10 can be characterized by a CPO reactor temperature of less than 1200, 1100, or 1000°C.

The CPO reactor 10 can be characterized by a CPO effluent temperature (e.g., target CPO effluent temperature) of greater than or equal to about 300 °C, about 600 °C, about 700 °C, about 750 °C, about 800 °C, or about 850 °C, alternatively from about 300 °C to about 1,600 °C, alternatively from about 600 °C to about 1,400 °C, alternatively from about 600 °C to about 1,300 °C, alternatively from about 700 °C to about 1,200 °C, alternatively from about 750 °C to about 1,150 °C, alternatively from about 800 °C to about 1,125 °C, or alternatively from about 850 °C to about 1,100 °C*.*

In embodiments, the CPO reactor 10 can be characterized by any suitable reactor temperature and/or catalyst bed temperature. For example, the CPO reactor 10 can be characterized by a reactor temperature and/or catalyst bed temperature of greater than or equal to about 300 °C, about 600 °C, about 700 °C, about 750 °C, about 800 °C, or about 850 °C, alternatively from about 300 °C to about 1,600 °C, , alternatively from about 600 °C to about 1,400 °C, alternatively from about 600 °C to about 1,300 °C, alternatively from about 700 °C to about 1,200 °C, alternatively from about 750 °C to about 1,150 °C, alternatively from about 800 °C to about 1,125 °C, or alternatively from about 850 °C to about 1,100 °C.

The CPO reactor 10 can be operated under any suitable temperature profile that can provide for a hydrogen-lean syngas as described herein. The CPO reactor 10 can be operated under adiabatic conditions, non-adiabatic conditions, isothermal conditions, near-isothermal conditions, autothermal conditions, etc. For purposes of the disclosure herein, the term "non-adiabatic conditions" refers to process conditions wherein a reactor is subjected to external heat exchange or transfer (e.g., the reactor is heated; or the reactor is cooled), which can be direct heat exchange and/or indirect heat exchange. As will be appreciated by one of skill in the art, and with the help of this disclosure, the terms "direct heat exchange" and "indirect heat exchange" are known to one of skill in the art. By contrast, the term "adiabatic conditions" refers to process conditions wherein a reactor is not subjected to external heat exchange (e.g., the reactor is not heated; or the reactor is not cooled). Generally, external heat exchange implies an external heat exchange system (e.g., a cooling system; a heating system) that requires energy input and/or output. External heat transfer can also result from heat loss from the catalyst bed (or reactor) due to radiation, conduction or convection. For example, this heat exchange from the catalyst bed can be to the external environment or to the reactor zones before and after the catalyst bed.

For purposes of the disclosure herein, the term "isothermal conditions" refers to process conditions (e.g., CPO operational parameters) that allow for a substantially constant temperature of the reactor and/or catalyst bed (e.g., isothermal temperature) that can be defined as a temperature that varies by less than about ± 10 °C, about ± 9 °C, about ± 8 °C, about + 7 °C, about ± 6 °C, about ± 5 °C, about ± 4 °C, about ± 3 °C, about ± 2 °C, or about ± 1 °C across the reactor and/or catalyst bed, respectively. Further, for purposes of the disclosure herein, the term "isothermal conditions" comprise a temperature variation of less than about ± 10 °C across the reactor and/or catalyst bed. In embodiments, the CPO reactor 10 can be operated under any suitable operational parameters that can provide for isothermal conditions.

For purposes of the disclosure herein, the term "near-isothermal conditions" refers to process conditions (e.g., CPO operational parameters) that allow for a fairly constant temperature of the reactor and/or catalyst bed (e.g., near-isothermal temperature), which can be defined as a temperature that varies by less than about ± 100 °C, about ± 90 °C, about ± 80 °C, about ± 70 °C, about ± 60 °C, about + 50 °C, about ± 40 °C, about ± 30 °C, about ± 20 °C, about ± 10 °C, about ± 9 °C, about ± 8 °C, about ± 7 °C, about ± 6 °C, about ± 5 °C, about ± 4 °C, about ± 3 °C, about ± 2 °C, or about ± 1 °C across the reactor and/or catalyst bed, respectively. In some embodiments, near-isothermal conditions allow for a temperature variation of less than about ± 50 °C, alternatively less than about + 25 °C, or alternatively less than about ± 10 °C across the reactor and/or catalyst bed. Further, for purposes of the disclosure herein, the term "near-isothermal conditions" is understood to include "isothermal" conditions. Furthermore, for purposes of the disclosure herein, the term "near-isothermal conditions" refers to process conditions that comprise a temperature variation of less than about ± 100 °C across the reactor and/or catalyst bed. In embodiments, a process as disclosed herein can comprise conducting the CPO reaction under near-isothermal conditions to produce the hydrogen-lean syngas, wherein the near-isothermal conditions comprise a temperature variation of less than about ± 100 °C across the reactor and/or catalyst bed. In embodiments, the CPO reactor 10 can be operated under any suitable operational parameters that can provide for near-isothermal conditions. Near-isothermal conditions can be provided by a variety of process and catalyst variables, such as temperature (e.g., heat exchange or heat transfer), pressure, gas flow rates, reactor configuration, catalyst bed configuration, catalyst bed composition, reactor cross sectional area, feed gas staging, feed gas injection, feed gas composition, and the like, or combinations thereof. Generally, and without wishing to be limited by theory, the terms "heat transfer" or "heat exchange" refer to thermal energy being exchanged or transferred between two systems (e.g., two reactors, such as a CPO reactor and a cracking reactor), and the terms "heat transfer" or "heat exchange" are used interchangeably for purposes of the disclosure herein.

In some embodiments, achieving a target CPO effluent temperature and/or near-isothermal conditions can be provided by heat exchange or heat transfer. The heat exchange can comprise heating the reactor; or cooling the reactor. In embodiments, achieving a target CPO effluent temperature and/or near-isothermal conditions can be provided by cooling the reactor. In another embodiment, achieving a target CPO effluent temperature and/or near-isothermal conditions can be provided by heating the reactor.

In some embodiments, achieving a target CPO effluent temperature and/or near-isothermal conditions can be provided by direct heat exchange and/or indirect heat exchange. As will be appreciated by one of skill in the art, and with the help of this disclosure, the terms "direct heat exchange" and "indirect heat exchange" are known to one of skill in the art. The heat exchange can comprise external heat exchange, external coolant fluid cooling, reactive cooling, liquid N₂ cooling, cryogenic cooling, electric heating, electric arc heating, microwave heating, radiant heating, natural gas combustion, solar heating, infrared heating, use of a diluent in the CPO reactant mixture, and the like, or combinations thereof. For example, reactive cooling can be effected by carrying out an endothermic reaction in a cooling coil/jacket associated with (e.g., located in) the reactor.

In some embodiments, achieving a target CPO effluent temperature and/or near-isothermal conditions can be provided by removal of process heat from the CPO reactor. In other embodiments, achieving a target CPO effluent temperature and/or near-isothermal conditions can be provided by supplying heat to the CPO reactor. As will be appreciated by one of skill in the art, and with the help of this disclosure, a CPO reactor may need to undergo both heating and cooling in order to achieve a target CPO effluent temperature and/or near-isothermal conditions.

In embodiments, the heat exchange or heat transfer can comprise introducing a cooling agent, such as a diluent, into the reactor (e.g., CPO reactor 10), to decrease the reactor temperature and/or the catalyst bed temperature, while increasing a temperature of the cooling agent and/or changing the phase of the cooling agent. The cooling agent can be reactive or non-reactive. The cooling agent can be in liquid state and/or in vapor state. As will be appreciated by one of skill in the art, and with the help of this disclosure, the cooling agent can act as a flammability retardant; for example by reducing the temperature inside the reactor, by changing the gas mixture composition, by reducing the combustion of hydrocarbons to CO₂; etc.

In some embodiments, the CPO reactant mixture 5 can further comprise a diluent, wherein the diluent contributes to achieving a target CPO effluent temperature and/or near-isothermal conditions via heat exchange, as disclosed herein. The diluent can comprise water, steam, inert gases (e.g., argon (Ar)), N₂, CO₂, and the like, or combinations thereof. Generally, the diluent is inert with respect to the CPO reaction, e.g., the diluent does not participate in the CPO reaction. However, and as will be appreciated by one of skill in the art, and with the help of this disclosure, some diluents (e.g., water, steam, CO₂, etc.) might undergo chemical reactions other than the CPO reaction within the reactor, and can change the composition of the resulting hydrogen-lean syngas 15, as will be described in more detail later herein; while other diluents (e.g., N₂, Ar) might not participate in reactions that change the composition of the resulting hydrogen-lean syngas 15. As will be appreciated by one of skill in the art, and with the help of this disclosure, the diluent can be used to vary the composition of resulting hydrogen-lean syngas 15. The diluent can be present in CPO reactant mixture 5 in any suitable amount.

The CPO reactor 10 can be characterized by a CPO pressure (e.g., reactor pressure measured at the reactor exit or outlet) of greater than or equal to about 1 barg, about 10 barg, about 20 barg, about 25 barg, about 30 barg, about 35 barg, about 40 barg, or about 50 barg, alternatively less than about 30 barg, about 25 barg, about 20 barg, or about 10 barg, alternatively from about 1 barg to about 90 barg, alternatively from about 1 barg to about 70 barg, alternatively from about 1 barg to about 40 barg, alternatively from about 1 barg to about 30 barg, alternatively from about 1 barg to about 25 barg, alternatively from about 1 barg to about 20 barg, alternatively from about 1 barg to about 10 barg, alternatively from about 20 barg to about 90 barg, alternatively from about 25 barg to about 85 barg, or alternatively from about 30 barg to about 80 barg.

The CPO reactor 10 can be characterized by a CPO contact time of from about 0.001 milliseconds (ms) to about 5 seconds (s), alternatively from about 0.001 ms to about 1 s, alternatively from about 0.001 ms to about 100 ms, alternatively from about 0.001 ms to about 10 ms, alternatively from about 0.001 ms to about 5 ms, or alternatively from about 0.01 ms to about 1.2 ms. Generally, the contact time of a reactor comprising a catalyst refers to the average amount of time that a compound (e.g., a molecule of that compound) spends in contact with the catalyst (e.g., within the catalyst bed), e.g., the average amount of time that it takes for a compound (e.g., a molecule of that compound) to travel through the catalyst bed. In some embodiments, the CPO reactor 10 can be characterized by a contact time of from about 0.001 ms to about 5 ms, or alternatively from about 0.01 ms to about 1.2 ms.

All of the CPO operational parameters disclosed herein are applicable throughout all of the embodiments disclosed herein, unless otherwise specified. As will be appreciated by one of skill in the art, and with the help of this disclosure, each CPO operational parameter can be adjusted to provide for a hydrogen-lean syngas as described herein. For example, the CPO operational parameters can be adjusted to provide for an increased H₂ content of the syngas, so long as the H₂/CO molar ratio remains in the desired range (e.g., from about 0.8 to about 1.6). As another example, the CPO operational parameters can be adjusted to provide for a decreased CO₂ content of the hydrogen-lean syngas 15. As yet another example, the CPO operational parameters can be adjusted to provide for a decreased unreacted hydrocarbons (e.g., unreacted CH₄) content of the hydrogen-lean syngas 15.

The embodiments, CPO reactor 10 is characterized by at least one CPO operational parameter selected from the group consisting of a CPO reactant mixture temperature of from about 100 °C to about 500 °C; a CPO pressure of from about 20 barg to about 80 barg; a CPO contact time of from about 0.001 milliseconds (ms) to about 5 seconds (s); a carbon to oxygen (C/O) molar ratio in the CPO reactant mixture of from about 0.5:1 to about 3:1, wherein the C/O molar ratio refers to the total moles of carbon (C) in the hydrocarbons in the reactant mixture divided by the total moles of O₂ in the reactant mixture; a steam to carbon (S/C) molar ratio in the CPO reactant mixture of less than about 0.6:1, wherein the S/C molar ratio refers to the total moles of water in the reactant mixture divided by the total moles of C in the hydrocarbons in the reactant mixture; a CO₂ to carbon (CO₂/C) molar ratio in the CPO reactant mixture of equal to or greater than about 0.5:1, wherein the CO₂/C molar ratio refers to the total moles of CO₂ in the reactant mixture divided by the total moles of C in the hydrocarbons in the reactant mixture; and combinations thereof.

In embodiments, CPO reactor 10 is characterized by at least one CPO operational parameter selected from the group consisting of a CPO reactant mixture temperature of from about 100 °C to about 500 °C; a CPO pressure of from about 25 barg to about 80 barg; a CPO contact time of from about 0.001 milliseconds (ms) to about 5 seconds (s); a C/O molar ratio in the CPO reactant mixture of from about 0.5:1 to about 2:1, wherein the C/O molar ratio refers to the total moles of carbon (C) in the hydrocarbons in the reactant mixture divided by the total moles of O₂ in the reactant mixture; a S/C molar ratio in the CPO reactant mixture of less than about 0.25:1, wherein the S/C molar ratio refers to the total moles of water (H₂O) in the reactant mixture divided by the total moles of carbon (C) in the hydrocarbons in the reactant mixture; a CO₂ to carbon (CO₂/C) molar ratio in the CPO reactant mixture of equal to or greater than about 0.5:1, wherein the CO₂/C molar ratio refers to the total moles of CO₂ in the reactant mixture divided by the total moles of carbon (C) in the hydrocarbons in the reactant mixture; and combinations thereof.

The CPO reaction is an exothermic reaction (e.g., heterogeneous catalytic reaction; exothermic heterogeneous catalytic reaction) that is generally conducted in the presence of a CPO catalyst comprising a catalytically active metal, i.e., a metal active for catalyzing the CPO reaction. The catalytically active metal can comprise a noble metal (e.g., Pt, Rh, Ir, Pd, Ru, Ag, and the like, or combinations thereof); a non-noble metal (e.g., Ni, Co, V, Mo, P, Fe, Cu, and the like, or combinations thereof); rare earth elements (e.g., La, Ce, Nd, Eu, and the like, or combinations thereof); oxides thereof; and the like; or combinations thereof. Generally, a noble metal is a metal that resists corrosion and oxidation in a water-containing environment. As will be appreciated by one of skill in the art, and with the help of this disclosure, the components of the CPO catalyst (e.g., metals such as noble metals, non-noble metals, rare earth elements) can be either phase segregated or combined within the same phase.

In embodiments, the CPO catalysts suitable for use in the present disclosure can be supported catalysts and/or unsupported catalysts. In some embodiments, the supported catalysts can comprise a support, wherein the support can be catalytically active (e.g., the support can catalyze a CPO reaction). For example, the catalytically active support can comprise a metal gauze or wire mesh (e.g., Pt gauze or wire mesh); a catalytically active metal monolithic catalyst; etc. In other embodiments, the supported catalysts can comprise a support, wherein the support can be catalytically inactive (e.g., the support cannot catalyze a CPO reaction), such as SiO₂; silicon carbide (SiC); alumina; a catalytically inactive monolithic support; etc. In yet other embodiments, the supported catalysts can comprise a catalytically active support and a catalytically inactive support.

In some embodiments, a CPO catalyst can be wash coated onto a support, wherein the support can be catalytically active or inactive, and wherein the support can be a monolith, a foam, an irregular catalyst particle, etc.

In some embodiments, the CPO catalyst can be a monolith, a foam, a powder, a particle, etc. Nonlimiting examples of CPO catalyst particle shapes suitable for use in the present disclosure include cylindrical, discoidal, spherical, tabular, ellipsoidal, equant, irregular, cubic, acicular, and the like, or combinations thereof.

In some embodiments, the support comprises an inorganic oxide, alpha, beta or theta alumina (Al₂O₃), activated Al₂O₃, silicon dioxide (SiO₂), titanium dioxide (TiO₂), magnesium oxide (MgO), zirconium oxide (ZrO₂), lanthanum (III) oxide (La₂O₃), yttrium (III) oxide (Y₂O₃), cerium (IV) oxide (CeO₂), zeolites, ZSM-5, perovskite oxides, hydrotalcite oxides, and the like, or combinations thereof.

Without limitation, CPO processes, CPO reactors, CPO catalysts, and CPO catalyst bed configurations suitable for use in the present disclosure are described in more detail in U.S. Provisional Patent Application No. 62/522,910 filed June 21, 2017 (International Application No. PCT/IB2018/054475 filed June 18, 2018) and entitled "Improved Reactor Designs for Heterogeneous Catalytic Reactions;" and U.S. Provisional Patent Application No. 62/521,831 filed June 19, 2017 (International Application No. PCT/IB2018/054470 filed June 18, 2018) and entitled "An Improved Process for Syngas Production for Petrochemical Applications;" each of which is hereby incorporated herein by reference in its entirety for purposes not contrary to this disclosure.

In embodiments, the CPO catalyst can be characterized by a catalyst productivity variation within about + 20%, about + 17.5%, about + 15%, about + 12.5%, about + 10%, about + 7.5%, about + 5%, about + 2.5%, or about + 1% of a target catalyst productivity over a time period of equal to or greater than about 500 hours (h), about 1,000 h, about 2,500 h, about 5,000 h, about 7,500 h, or about 10,000 h; wherein catalyst productivity is defined as the amount of hydrogen-lean syngas 15 (e.g., CPO reactor effluent) recovered from CPO reactor 10 divided by the amount of hydrocarbons introduced to CPO reactor 10 in CPO reactant mixture 5. As will be appreciated by one of skill in the art, and with the help of this disclosure, and without wishing to be limited by theory, catalyst productivity is a quantitative measure of catalyst activity, wherein the catalyst activity refers to the ability of a catalyst (e.g., CPO catalyst) to increase the rate of a chemical reaction (e.g., CPO reaction) under a given set of reaction conditions (e.g., CPO operational parameters). For purposes of the disclosure herein, a CPO catalyst having a productivity variation greater than about + 20% can be referred to as a "spent CPO catalyst" (as opposed to an active CPO catalyst). As used herein, the target catalyst productivity is associated with an active CPO catalyst (e.g., fresh CPO catalyst and/or regenerated CPO catalyst). For purposes of the disclosure herein, the term "fresh CPO catalyst" refers to a CPO catalyst that has not been used in a CPO process. As will be appreciated by one of skill in the art, and with the help of this disclosure, an active CPO catalyst displays optimum (e.g., maximum) catalyst activity with respect to a chemical reaction (e.g., CPO reaction) under a given set of reaction conditions (e.g., CPO operational parameters). Further, and as will be appreciated by one of skill in the art, and with the help of this disclosure, the target catalyst productivity is the maximum catalyst productivity of an active CPO catalyst (e.g., fresh CPO catalyst and/or regenerated CPO catalyst) under a given set of reaction conditions (e.g., CPO operational parameters). Furthermore, and as will be appreciated by one of skill in the art, and with the help of this disclosure, the terms "catalyst productivity" and "target catalyst productivity" are used in the context of steady-state operation of the CPO reactor (e.g., CPO reactor 10).

As will be appreciated by one of skill in the art, and with the help of this disclosure, catalyst activity (e.g., CPO catalyst activity) can vary (e.g., decay, decrease) over time, for a variety of reasons, such as poisoning (e.g., feed contaminants), fouling (e.g., coking by carbon produced by cracking/condensation/decomposition reactions of hydrocarbon reactants, intermediates, and/or products), thermal degradation (e.g., collapse of support structure, solid-state reactions, attrition), active component leaching, migration of active components within and/or outside catalyst particles, side reactions, attrition/crushing, and the like, or combinations thereof. Decay in catalyst activity leads to spent catalysts (e.g., spent CPO catalysts). In embodiments, spent catalysts can be regenerated and returned to a production process, as will be described in more detail later herein.

In embodiments, a portion of the hydrocarbons (e.g., methane) in the CPO reactant mixture 5 can undergo a thermal decomposition reaction to carbon (C) and H₂, for example as represented by equation (2):

CH₄ → C + 2 H₂ (2)

The decomposition reaction of hydrocarbons, such as CH₄, is facilitated by elevated temperatures, and increases the H₂ content in the hydrogen-lean syngas. However, the carbon produced by the decomposition reaction of hydrocarbons (e.g., a decomposition reaction as represented by equation (2)) can lead to coking of the CPO catalyst via carbon deposition onto the CPO catalyst, thereby producing a spent CPO catalyst. As will be appreciated by one of skill in the art, and with the help of this disclosure, and without wishing to be limited by theory, while the percentage of hydrocarbons in the CPO reactant mixture 5 that undergoes a decomposition reaction (e.g., a decomposition reaction as represented by equation (2)) increases with increasing the C/O molar ratio in the CPO reactant mixture 5, a portion of hydrocarbons can undergo a decomposition reaction to C and H₂ even at relatively low C/O molar ratios in the CPO reactant mixture 5 (e.g., a C/O molar ratio in the CPO reactant mixture 5 of less than about 1:1). Further, and as will be appreciated by one of skill in the art, and with the help of this disclosure, the quality of the hydrocarbon feed to the CPO reactor 10 can influence coking. For example, higher hydrocarbons (e.g., hydrocarbons having equal to or greater than 2 C atoms, C₂₊) can produce more coke than CH₄, owing to having a higher carbon content than CH₄.

In an aspect, CPO reactant mixture 5 can further comprise a diluent, such as water and/or steam, and CO₂. CPO reactor 10 can be operated under any suitable operational conditions (e.g., CPO operational parameters) that can provide for a syngas with a desired composition (e.g., desired H₂/CO molar ratio; desired CO₂ content; etc.); for example, the CPO reactor 10 can be operated with introducing water and/or steam, and CO₂ to CPO reactor 10.

When carbon is present in the reactor (e.g., coke; C produced as a result of a decomposition reaction as represented by equation (2)), water and/or steam diluent can react with the carbon and generate additional CO and H₂, for example as represented by equation (3):

C + H₂O 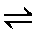 CO + H₂ (3)

As will be appreciated by one of skill in the art, and with the help of this disclosure, the presence of water and/or steam in the CPO reactor 10 can decrease the amount of coke in the CPO reactor 10 (e.g., the amount of coke deposited on the CPO catalyst, the amount of spent CPO catalyst present in the CPO reactor 10), thereby providing for maintaining the catalyst productivity.

Further, and as will be appreciated by one of skill in the art, and with the help of this disclosure, water and/or steam can be used to vary the composition of the resulting hydrogen-lean syngas 15 in the CPO reactor effluent. Steam can react with methane, for example as represented by equation (4):

CH₄ + H₂O 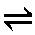 CO + 3 H₂ (4)

In an aspect, a diluent comprising water and/or steam can increase a hydrogen content of the resulting hydrogen-lean syngas 15 in the CPO reactor effluent. For example, in aspects where CPO reactant mixture 5 comprises water and/or steam diluent, the resulting hydrogen-lean syngas 15 in the CPO reactor effluent can be characterized by a hydrogen to CO molar ratio that is increased when compared to a hydrogen to CO molar ratio of a syngas produced by an otherwise similar process conducted with a reactant mixture comprising hydrocarbons and O₂ without the water and/or steam diluent. Without wishing to be limited by theory, the reforming reaction (e.g., as represented by eqn. (4)) is an endothermic reaction. The reforming reaction as represented by eqn. (4) can remove a portion of the process heat (e.g., heat produced by exothermic CPO reaction, for example as represented by eqn. (1)).

In the presence of water and/or steam in the CPO reactor 10, CO can react with the water and/or steam to form CO₂ and H₂ via a water-gas shift (WGS) reaction, for example as represented by equation (5):

CO + H₂O 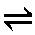 CO₂ + H₂ (5)

While the WGS reaction can increase the H₂/CO molar ratio of the syngas produced by the CPO reactor 10, it also produces CO₂.

Injection of steam and/or water can thus be utilized, in embodiments, to enhance the production of CO and reduce the quality of the hydrogen-lean syngas 15. thus providing a hydrogen-lean syngas 15 having a lower molar ratio of H₂/CO. Injection of steam and/or water can also help maintain CPO catalyst activity. In embodiments, the CPO reactant mixture 5 can be characterized by a steam to carbon (S/C) and/or a steam to CH₄ (S/CH₄) molar ratio of less than or equal to 1.0, 0.9, 0.8, 0.7, 0.6, 0.5, 0.4, 0.3. 0.2, or in a range of from about 0.1, 0.2, or 0.3 to about 0.4, 0.5, 0.6, ,0.7, 0.8, 0.9, or 1, from about 0.2 to about 1, from about 0.2 to about 0.6, or from about 0.2 to about 0.5. In embodiments, the CPO reactor 10 can be operated at an S/C molar ratio ) and/or a steam to CH₄ (S/CH₄) molar ratio in the CPO reactant mixture 5 of less than about 0.6:1, alternatively less than about 0.5:1, alternatively less than about 0.4:1, alternatively less than about 0.3:1, alternatively less than about 0.2:1, alternatively less than about 0.1:1, alternatively from about 0.01:1 to less than about 0.6:1, alternatively from about 0.05:1 to about 0.6:1, alternatively from about 0.1:1 to about 0.5:1, alternatively from about 0.15:1 to about 0.6:1, or alternatively from about 0.2:1 to about 0.6:1. As will be appreciated by one of skill in the art, and with the help of this disclosure, the steam that is introduced to the CPO reactor for use as a diluent in a CPO reaction as disclosed herein is present in significantly smaller amounts than the amounts of steam utilized in steam reforming (e.g., SMR) processes, and as such, a process for producing syngas as disclosed herein can yield a (e.g., hydrogen-lean) syngas with lower amounts of hydrogen when compared to the amounts of hydrogen in a syngas produced by steam reforming.

The S/C molar ratio in the CPO reactant mixture 5 can be adjusted based on the desired CPO effluent temperature (e.g., target CPO effluent temperature) in order to adjust the H₂ content of the produced syngas (e.g., syngas 15). As will be appreciated by one of skill in the art, and with the help of this disclosure, the reaction (4) that consumes steam in the CPO reactor may be less preferable over the water-gas shift (WGS) reaction (5) in the CPO reactor 10, as reaction (4) allows for increasing the H₂ content of the produced syngas (e.g., syngas 15), as well as the M ratio of the produced syngas (e.g., syngas 15), wherein the M ratio is a molar ratio defined as (H₂-CO₂)(CO+CO₂). Further, and as will be appreciated by one of skill in the art, and with the help of this disclosure, reaction (5) converts water and CO to both H₂ and CO₂.

Without wishing to be limited by theory, the presence of water and/or steam in the CPO reactor 10 changes the flammability of the CPO reactant mixture 5, thereby providing for a wider practical range of C/O molar ratios in the CPO reactant mixture 5. Further, and without wishing to be limited by theory, the presence of water and/or steam in the CPO reactor 10 allows for the use of lower C/O molar ratios in the CPO reactant mixture 5. Furthermore, and without wishing to be limited by theory, the presence of water and/or steam in the CPO reactor 10
allows for operating the CPO reactor 10 at relatively high pressures.

As will be appreciated by one of skill in the art, and with the help of this disclosure, the introduction of water and/or steam in the CPO reactor 10 can lead to increasing the amount of unreacted hydrocarbons in the syngas 15. Further, as will be appreciated by one of skill in the art, and with the help of this disclosure, some downstream chemical synthesis processes tolerate limited amounts of unreacted hydrocarbons in the syngas.

In some aspects, the hydrogen-lean syngas 15 can comprise less than about 7.5 mol%, alternatively less than about 5 mol%, or alternatively less than about 2.5 mol% hydrocarbons (e.g., unreacted hydrocarbons, unreacted CH₄). In such aspects, the syngas 15 can be produced in a CPO process that employs water and/or steam.

CO₂ is introduced into the CPO reactor 10 (e.g., via line 7, 7A, and/or 7B). Since O₂ is present in the CPO reactant mixture 5, the carbon present in the reactor (e.g., coke; C produced as a result of a decomposition reaction as represented by eqn. (2)) can also react with O₂, for example as represented by eqn. (6):

C + O₂ →CO₂ (6)

When carbon is present in the reactor (e.g., coke; C produced as a result of a decomposition reaction as represented by equation (2)), CO₂ (e.g., introduced to the CPO reactor 10 as part of the CPO reactant mixture 5 and/or produced by the reaction represented by eqn. (6)) can react with the carbon, for example as represented by eqn. (7):

C + CO₂ ↔ 2 CO (7)

thereby decreasing the amount of CO₂ and increasing the amount of CO in the resulting hydrogen-lean syngas 15. The use of reactant mixtures 5 comprising higher hydrocarbons (e.g., C₂₊) can lead to the formation of a greater amount of coke, and thus lead to an enrichment of CO and a reduced H₂/CO molar ratio in the hydrogen-lean syngas 15. As will be appreciated by one of skill in the art, and with the help of this disclosure, the presence of CO₂ in the CPO reactor 10 can decrease the amount of coke in the CPO reactor 10 (e.g., the amount of coke deposited on the CPO catalyst, the amount of spent CPO catalyst present in the CPO reactor 10), thereby providing for maintaining the catalyst productivity. Injection of CO₂ also provides for an enhancement in carbon efficiency, because the carbon in the CO₂ is converted to additional CO. As a result, more CO will be produced per MMBTU of reactant feed (e.g., natural gas) according to embodiments of this disclosure. This additional CO can contribute to an increase in chemical product through put (e.g., an increase in the throughput of acetic acid in the process of Figure 1 or DME in the process of Figure 2) the same flowrate of reactant feed (e.g., natural gas).

Further, CO₂ can react with CH₄ in a dry reforming reaction, for example as represented by eqn. (8):

CH₄ + CO₂ ↔ CO +2H₂ (8)

thereby decreasing the amount of CO₂ in the resulting syngas in the CPO reactor effluent 15. Without wishing to be limited by theory, the dry reforming reaction (e.g., as represented by equation (8)) is an endothermic reaction (e.g., highly endothermic reaction). The dry reforming reaction can remove a portion of the process heat (e.g., heat produced by the exothermic CPO reaction, for example as represented by equation (1)).

In embodiments, a diluent comprising CO₂ can increase a CO content of the resulting hydrogen lean syngas 15. The hydrogen-lean syngas 15 can be characterized by a hydrogen to CO molar ratio that is decreased when compared to a hydrogen to CO molar ratio of a syngas produced by an otherwise similar process conducted with a reactant mixture comprising hydrocarbons and O₂ without the CO₂ diluent. Without wishing to be limited by theory, CO₂ can react with coke inside the CPO reactor 10 and generate additional CO, for example as represented by equation (7). Further, and without wishing to be limited by theory, CO₂ can participate in a dry reforming of methane reaction, thereby generating additional CO and H₂, for example as represented by equation (8). Dry reforming of methane is generally accompanied by a reaction between CO₂ and hydrogen which results in the formation of additional CO and water.

In embodiments, the CPO reactant mixture 5 can comprise CO₂ in an amount effective to provide for less than about 7 mol%, alternatively less than about 6 mol%, alternatively less than about 5 mol%, alternatively from about 0.1 mol% to about 7 mol%, alternatively from about 0.25 mol% to about 6 mol%, or alternatively from about 0.5 mol% to about 5 mol% CO₂ in the hydrogen-lean syngas 15, based on the total mol% of the syngas. The CO₂ of the CPO reactant mixture 5 can be CO₂ from natural gas sources, wherein the CO₂ is introduced to the CPO reactor 10 with the hydrocarbons; and/or additional or supplemental CO₂. for example CO₂ recovered as a process stream and recycled to CPO reactor 10 (e.g., via CO₂ stream 7, 7A, and/or 7B).

In embodiments, the conversion of hydrocarbons in the CPO reactor 10 is greater than the conversion of hydrocarbons in a CPO reactor in an otherwise similar process that produces a hydrogen-lean syngas from hydrocarbons comprising a reduced amount of higher hydrocarbons (e.g., C₂₊ hydrocarbons). For example, in embodiments, the conversion of hydrocarbons in the CPO reactor 10 of a CPO reactant mixture 5 comprising greater than or equal to about 5, 4, or 3 mol C₂₊ alkanes is greater than the conversion of hydrocarbons in a CPO reactor in an otherwise similar process that produces a hydrogen-lean syngas from a CPO reactant mixture 5 comprising less than about 5, 4, or 3 mol C₂₊ alkanes, respectively.

In embodiments, no further adjustment of the H₂/CO molar ratio of the hydrogen-lean syngas 15 is provided prior to downstream synthesis of DME or acetic acid. That is, the herein disclosed CPO system and method can be utilized to produce a hydrogen-lean syngas 15 having a H₂/CO molar ratio suitable for downstream production of acetic acid or DME (e.g., an H₂/CO molar ratio on the range of from about 0.8 to 1.6). Thus, in embodiments, a process as disclosed herein excludes a step of a step of introducing at least a portion of the hydrogen-lean syngas 15 to a hydrogen recovery unit to decrease the amount of hydrogen in the hydrogen-lean syngas 15. Thus, in embodiments, a process as disclosed herein does not comprise altering the H₂/CO molar ratio of the hydrogen-lean 15 between the CPO reactor 10 and the downstream synthesis apparatus. Thus, in embodiments, a chemical synthesis system as disclosed herein does not comprise apparatus (e.g., a hydrogen removal unit, PSA) for altering the H₂/CO molar ratio of the hydrogen-lean syngas 15 between the CPO reactor 10 and the downstream synthesis apparatus (e.g., either the acetic acid synthesis apparatus described herein below with reference to the embodiment of Figure 1 or the DME synthesis apparatus of the embodiment of Figure 2).

In embodiments, a process as disclosed herein comprises no adjusting of the H₂/CO molar ratio of the hydrogen-lean syngas 15 prior to the utilizing the hydrogen-lean syngas 15 in downstream chemical synthesis. Thus, in embodiments, a chemical synthesis system as disclosed herein comprises no apparatus for adjusting the H₂/CO molar ratio of the hydrogen-lean syngas 15 prior to the downstream acetic acid or DME synthesis reactor.

In embodiments, a process as disclosed herein does not comprise removing a hydrogen stream from the hydrogen-lean syngas 15 prior to utilizing the hydrogen-lean syngas 15 in downstream acetic acid or DME synthesis. Thus, in embodiments, a chemical synthesis system as disclosed herein comprises no apparatus configured to remove a hydrogen stream from the hydrogen-lean syngas 15 between the CPO reactor 10 and the downstream synthesis apparatus (e.g., DME carbonylation unit 30 described hereinbelow with reference to the embodiment of Figure 1 or DME reactor 70 described hereinbelow with reference to the embodiment of Figure 2).

In embodiments, the CPO reactor 10 can produce the hydrogen lean syngas 15 at high pressures (e.g., greater than or equal to about 20, 25, 30, 35, 40, 45, 50 bar) that are required for downstream chemical (e.g., acetic acid, DME) synthesis, thus reducing the size of or eliminating the need for one or more compressors 20 to compress the hydrogen-lean syngas 15 prior to downstream chemical synthesis. Accordingly, the herein disclosed systems and processes for producing acetic acid or DME via a hydrogen-lean syngas obtained via CPO can, in embodiments, further reduce energy requirements for production of the acetic acid or DME.

In embodiments, the hydrogen-lean syngas 15 can have a CO₂ content of less than about 10 mol%, less than about 9 mol%, less than about 8 mol%, less than about 7 mol%, alternatively less than about 6 mol%, alternatively less than about 5 mol%, alternatively less than about 4 mol%, alternatively less than about 3 mol%, alternatively less than about 2 mol%, alternatively less than about 1 mol%, alternatively greater than about 0.1 mol%, alternatively greater than about 0.25 mol%, alternatively greater than about 0.5 mol%, alternatively from about 0.1 mol% to about 7 mol%, alternatively from about 0.25 mol% to about 6 mol%, or alternatively from about 0.5 mol% to about 5 mol%. For example, side reactions could lead to a hydrogen lean syngas 15 that has a CO₂ content of less than about 7 mol%, alternatively less than about 6 mol%, alternatively less than about 5 mol%, alternatively from about 0.1 mol% to about 7 mol%, alternatively from about 0.25 mol% to about 6 mol%, or alternatively from about 0.5 mol% to about 5 mol%. As noted hereinabove, the CO₂ concentration in the hydrogen-lean syngas 15 can be controlled via CO₂ injection (e.g., via CO₂ stream 7) and/or by changing the operating conditions of CPO reactor 10.

The amount of CO₂ in H₂-lean syngas 15 can be adjusted depending on the downstream application. In such applications, the amount of CO₂ in H₂-lean synthesis gas 15 can be adjusted as provided hereinabove.

In embodiments, the hydrogen-lean syngas 15 can be subjected to processing, such as the recovery of unreacted hydrocarbons, diluent, water, etc. In embodiments, water can be condensed and separated from the hydrogen-lean syngas 15, e.g., in a condenser. As understood, such processing for the removal of hydrocarbons, diluent, water, etc. will not alter an H₂/CO molar ratio of the hydrogen-lean syngas stream 15. In embodiments, a process as disclosed herein can further comprise: (i) recovering at least a portion of the unreacted hydrocarbons from the hydrogen-lean syngas 15 to yield recovered hydrocarbons, and (ii) recycling at least a portion of the recovered hydrocarbons to the CPO reactor 10. As will be appreciated by one of skill in the art, and with the help of this disclosure, although fairly high conversions can be achieved in CPO processes (e.g., conversions of greater than or equal to about 90%), the unconverted hydrocarbons could be recovered and recycled back to the CPO reactor 10.

In embodiments, a process as disclosed herein further comprises producing acetic acid or DME from at least a portion of the hydrogen-lean syngas, as selectivity of DME and acetic acid is enhanced by syngas having an H₂/CO molar ratio of about 1:1. The term "unit" (for example in DME carbonylation "unit" or methanol synthesis "unit") refers to a unit that can comprise separation equipment in addition to the reactor. For clarity, such separation equipment is not depicted in Figures 1 or 2.

In embodiments, a process as disclosed herein further comprises producing acetic acid from at least a portion of the hydrogen-lean syngas 15. In embodiments, any system and method for producing acetic acid from the hydrogen-lean synthesis gas 15 can be utilized downstream of CPO reactor 10, as per this disclosure, to produce acetic acid. In embodiments, a process as disclosed herein comprises producing acetic acid from at least a portion of the hydrogen-lean syngas 15 via intermediate methanol synthesis. When producing hydrogen-lean syngas 15 for downstream acetic acid production, the CO₂ concentration in the hydrogen-lean syngas can be controlled (e.g., by CO₂ injection and/or changing the operating conditions of CPO reactor 10) to provide a small amount of CO₂ in the hydrogen-lean syngas 15, as such CO₂ will increase the formation of methanol, which is an intermediate in acetic acid synthesis, thus increasing the production of acetic acid. For example, small amounts of CO₂ may be desirable in the hydrogen-lean syngas 15 when feeding a downstream DME reactor 70 (as described hereinbelow with reference to the embodiment of Figure 2), wherein small amounts of CO₂ in the hydrogen-lean syngas 15 can be desirable to increase the production of methanol (which is an intermediate in the acetic acid production) and thus enhance DME synthesis.

In embodiments, a process as disclosed herein further comprises feeding at least a portion of the hydrogen-lean syngas 15 and dimethyl ether (DME) 51 to a DME carbonylation unit 30 to produce methyl acetate 35 and a hydrogen-enriched syngas; and feeding at least a portion of methyl acetate 35 and water 36 to a methyl acetate hydrolysis reaction zone 36 to produce acetic acid 65 and a methanol stream 45A. In embodiments, the hydrogen-enriched syngas 31 comprises hydrogen, CO, CO₂, and unreacted hydrocarbons, and is characterized by a H₂/CO molar ratio of from about 1.4, 1.5, 1.6, 1.7, or 1.8 to about 1.8, 1.9, 2.0, 2.1, or 2.2. The DME 51 can comprise DME 51A from a DME synthesis reaction zone 50 (described further hereinbelow) and/or DME 51B from another source.

Within DME carbonylation unit 30, DME 51 and CO from hydrogen-lean syngas 15 are converted to methyl acetate. Any suitable carbonylation catalyst can be utilized in DME carbonylation unit 30, and DME carbonylation unit 30 can be operated under any suitable DME carbonylation reaction conditions known to those of skill in the art. In other embodiments, methanol carbonylation to produce acetic acid occurs in a single step.

In embodiments, the CPO reactor 10 is characterized by a CPO pressure; the DME carbonylation unit 30 is characterized by a DME carbonylation pressure; and the CPO pressure is about the same as the DME carbonylation pressure. In alternative embodiments, the CPO reactor 10 is characterized by a CPO pressure, the DME carbonylation unit 30 is characterized by a DME carbonylation pressure that is greater than the CPO pressure, and at least a portion of the hydrogen-lean syngas 15 is compressed in a compressor 20 to yield a compressed syngas 15' such that the compressed syngas 15' is characterized by a pressure that is about the same as the DME carbonylation pressure prior to feeding of least a portion of the compressed syngas 15' to the DME carbonylation unit 30.

In embodiments, the size of compressor 20 is smaller than the size of a compressor used for compressing hydrogen-lean syngas in an otherwise similar process that (i) employs a hydrogen recovery unit for producing a hydrogen-lean syngas; (ii) produces a hydrogen-lean syngas from hydrocarbons comprising less than about 3 mol% C₂₊ alkanes; and/or (iii) does not produce the hydrogen-lean syngas by CPO.

Methyl acetate hydrolysis reaction zone 36 can be any reaction zone operable to produce acetic acid 65 and methanol 45A from methyl acetate 35 and water 36, and can be operated under any operating conditions suitable for hydrolyzing methyl acetate into methanol and acetic acid.

In embodiments, a method of producing acetic acid according to this disclosure further comprises feeding at least a portion of the hydrogen-enriched syngas 31 to a methanol synthesis unit 40 to produce another (i.e., a 'second') methanol stream 45B and a purge gas stream 41, wherein the purge gas stream comprises hydrogen, CO, CO₂, and unreacted hydrocarbons, and wherein at least a portion of the purge gas stream 41 is optionally used as fuel. Methanol synthesis unit 40 can comprise any suitable methanol synthesis reactor known in the art, and can be operated under reaction conditions known to those of skill in the art.

In embodiments, a process for producing acetic acid according to this disclosure further comprises: (1) feeding at least a portion of the (e.g., first) methanol stream 45A and/or at least a portion of the another (e.g., second) methanol stream 45B to a DME synthesis reaction zone 50 to produce a DME stream 51B; and (2) feeding at least a portion of the DME stream 51A to the DME carbonylation unit 30.

In embodiments, DME synthesis reaction zone 50 is operable to produce DME via dehydration of methanol. In embodiments, a common reactor comprises both the methyl acetate hydrolysis reaction zone 60 and the DME synthesis reaction zone 50. In embodiments, at least a portion of the water produced in DME synthesis reaction zone 50 is utilized as the water feed 36 to methyl acetate hydrolysis zone 60. In embodiments, methanol synthesis from syngas 31 and dehydration of the methanol (45A and/or 45B) to produce DME occur in a single process unit.

In embodiments, the amount of acetic acid 65 produced is greater than the amount of acetic acid produced in an otherwise similar process that (i) employs a hydrogen recovery unit for producing a hydrogen-lean syngas; (ii) produces a hydrogen-lean syngas from hydrocarbons comprising less than about 3 mol% C₂₊ alkanes; and/or (iii) does not produce the hydrogen-lean syngas by CPO.

In embodiments, such as depicted in the embodiment of Figure 2, a process as disclosed herein further comprises producing DME from at least a portion of the hydrogen-lean syngas 15. In embodiments, a process as disclosed herein further comprises feeding at least a portion of the hydrogen-lean syngas 15 to a dimethyl ether (DME) reactor 70 to produce a DME reactor effluent 75 comprising DME, methanol, water, and CO₂.

The DME reactor 70 is characterized by a DME reactor pressure which can be the same or different from the CPO reactor pressure. When the DME pressure is greater than the CPO pressure, the method of producing DME can further comprise: (1) compressing at least a portion of the hydrogen-lean syngas 15 to yield a compressed syngas 15', wherein the pressure of the compressed syngas 15' is about the same as the pressure of DME reactor 70; and (2) feeding at least a portion of the compressed syngas 15' to the DME reactor 70.

DME reactor 70 can be any reactor known to be suitable for the conversion of hydrogen-lean syngas 15 (e.g., compressed hydrogen-lean syngas 15') into a DME reactor effluent comprising DME, water, methanol, and CO₂, and can be operated under any suitable reactor operating conditions known in the art. DME reactor 70 can comprise a two catalyst system whereby both methanol synthesis and dehydration of methanol to produce DME occur therein. The method of producing DME according to embodiments of this disclosure can further comprise separating at least a portion of the DME reactor effluent 75 into a DME stream 82, a methanol stream 81, a water stream 83, and a CO₂ stream 7B, and optionally recycling at least a portion of the methanol stream 81 to the DME reactor 70, and/or optionally recycling at least a portion of the CO₂ stream 7B to the CPO reactor 10.

In embodiments, a process for producing DME according to this disclosure provides for the production of an amount of DME 82 that is greater than the amount of DME produced in an otherwise similar process that (i) employs a H₂ recovery unit for producing a H₂-lean syngas; (ii) produces a H₂-lean syngas from hydrocarbons comprising less than about 3 mol C₂₊ alkanes; and/or (iii) does not produce the H₂-lean syngas by CPO.

In embodiments, a process as disclosed herein can advantageously display improvements in one or more process characteristics when compared to conventional processes.

As will be appreciated by one of skill in the art, and with the help of this disclosure, since the CPO reaction is exothermic, very little heat supply in the form of fuel combustion is needed (e.g., for pre-heating reactants in the reaction mixture 5 that is supplied to the CPO syngas generation section), when compared to conventional steam reforming. As such, the process for chemical synthesis utilizing CPO hydrogen-lean syngas as disclosed herein can advantageously generate less CO₂ through fuel burning, when compared to steam reforming.

The use of CPO reactant mixtures comprising higher hydrocarbons and CO₂ as described herein provides a high selectivity and thus increases the overall carbon efficiency of acetic acid or DME synthesis relative to conventional processes. Because CPO can be operated at higher pressures than conventional syngas syntheses (e.g., dry reforming) utilized to produce hydrogen-lean syngas, energy requirements (e.g., energy required for compressing of the hydrogen-lean syngas prior to downstream acetic acid or DME synthesis therefrom) can be reduced (or such compression eliminated) relative to the conventional processes.

The herein disclosed process for the synthesis of acetic acid provides for the production of a hydrogen enriched synthesis gas 31 (e.g., from DME carbonylation unit 30) that can be utilized directly for the production of DME in DME synthesis reaction zone 50 without adjustment of the H₂/CO molar ratio.

Additional advantages of the processes for the production methanol as disclosed herein can be apparent to one of skill in the alt viewing this disclosure.

### EXAMPLES

The embodiments having been generally described, the following examples are given as particular embodiments of the disclosure and to demonstrate the practice and advantages thereof. It is understood that the examples are given by way of illustration and are not intended to limit the specification or the claims in any manner.

Example 1. A syngas process was simulated as an equilibrium reactor in ASPEN. Figure 3 is a plot of the molar ratio of CO/H₂ in syngas from CPO as a function of reactor temperature without CO₂ injection in the reactant feed for CH₄O₂ molar ratios of 2.2 and 1.7, and pressures of 40 and 100 bar, which shows the CO/H₂ molar ratios which can be obtained in CPO subject to thermodynamic constraints at different temperatures of CPO reactor
10. Figure 4 is a plot of molar ratio of CO/H₂ in syngas from CPO as a function of reactor temperature with CO₂ injection for a reactant feed comprising a CO₂/CH₄ molar ratio of 0.5, CH₄O₂ molar ratios of 2.2 and 1.7, and pressures of 40 and 100 bar. Figure 5 is a plot of the CO/H₂ in syngas from CPO as a function of reactor temperature with CO₂ injection for a reactant feed comprising a CO₂/CH₄ molar ratio of 1, CH₄O₂ molar ratios of 2.2 and 1.7, and pressures of 40 and 100 bar.

As seen in Figure 3, above 900 °C and at a low CH₄O₂ molar ratio synthesis gas having H₂/CO molar ratios less than 2 can be produced. From Figure 4 and Figure 5. it is apparent that injecting CO₂ in the reactant feed expands the operability window of CPO to lower temperatures and higher CH₄O₂ molar ratios. As noted herein, injection of CO₂ also provides for an enhancement in carbon efficiency, because the carbon in the CO₂ is converted to additional CO. As a result more CO will be produced per MMBTU of reactant feed (e.g., natural gas) according to embodiments of this disclosure. This additional CO can contribute to an increase in chemical product (e.g., acetic acid or DME) throughput at the same flowrate of reactant feed (e.g., natural gas). As seen in Figures 3-5, the CPO reactor can produce the hydrogen-lean syngas at high pressures (e.g., greater than or equal to about 25, 30, 35, 40, 45, 50 bar) that are required for downstream chemical (e.g., acetic acid, DME) synthesis, thus reducing or eliminating the need for compression of the hydrogen-lean syngas 15 prior to the downstream synthesis of acetic acid or DME.

Figure 6 is a plot of the molar ratio of CO₂ to CO (CO₂/CO) in syngas from CPO as a function of reactor temperature without CO₂ injection for reactant feeds comprising a molar ratio of methane to O₂ (CH₄/O₂) of 2.2 or 1.7 and pressures of 40 or 100 bar. Figure 7 is a plot of the molar ratio of CO₂ to carbon (CO₂/CO) in syngas from CPO as a function of reactor temperature with CO₂ injection for a reactant feed comprising a molar ratio of CO₂ to methane (CO₂/CH₄) of 0.5, CH/O₂ molar ratios of 2.2 and 1.7, and pressures of 40 and 100 bar.

Figure 8 is a plot showing the molar ratio of CO to hydrogen (H₂/CO) in syngas from CPO as a function of conversion (%) and molar ratio of CO₂ to carbon (CO₂/C) in reactant feed (in legend) at a pressure of 30 bar and O₂ to carbon molar ratio (O₂/C) of 0.55. Figure 9 is a plot showing the molar ratio of CO to hydrogen (H₂/CO) in syngas from CPO as a function of the conversion (%) and the molar ratio of CO₂ to carbon (CO₂/C) in the reactant feed (in legend) at a pressure of 75 bar and an O₂/C molar ratio of 0.55. As can be seen from Figures 8 and 9, the molar ratio of CO₂/C needed to provide a H₂-lean CPO syngas having a H₂/CO molar ratio of 1 is reduced as pressure increases.

Figure 10 is a plot showing the molar ratio of CO to hydrogen (H₂/CO) in syngas from CPO as a function of the conversion (%) and the molar ratio of hydrocarbons having three carbons (C₃) to carbon (C₃/C) in the reactant feed (in legend) at a pressure of 75 bar, O₂ to carbon molar ratio (O₂/C) of 0.55, and CO₂ to carbon (CO₂/C) molar ratio of 0.25. Figure 1 1 is a plot showing the molar ratio of CO to hydrogen (H₂/CO) in syngas from CPO as a function of the conversion (%) and molar ratio of hydrocarbons having three carbons (C₃) to carbon (C₃/C) in reactant feed (in legend) at a pressure of 75 bar, O₂ to carbon molar ratio (O₂/C) of 0.55, and without CO₂ in reactant feed.

Figure 12 is a plot showing the molar ratio of CO to hydrogen (H₂/CO) in syngas from CPO as a function of the conversion (%) and molar ratio of hydrocarbons having two carbons (C₂) to carbon (C₂/C) in reactant feed (in legend) at a pressure of 75 bar, an O₂ to carbon molar ratio (O₂/C) of 0.55, and a CO₂ to carbon (CO₂/C) molar ratio of 0.25. Figure 13 is a plot the molar ratio of CO to hydrogen (H₂/CO) in syngas from CPO as a function of the conversion (%) and the molar ratio of hydrocarbons having two carbons (C₂) to carbon (C₂/C) in the reactant feed (in legend) at a pressure of 75 bar, an O₂ to carbon molar ratio (O₂/C) of 0.55, and without CO₂ in the reactant feed.

Figure 14 is a plot showing the molar ratio of CO to hydrogen (H₂/CO) in syngas from CPO as a function of the conversion (%) and the molar ratio of hydrocarbons having four carbons (C₄) to carbon (C₄/C) in the reactant feed (in legend) at a pressure of 75 bar, an O₂ to carbon molar ratio (O₂/C) of 0.55, and a CO₂ to carbon (CO₂/C) molar ratio of 0.25. Figure 15 is a plot showing the molar ratio of CO to hydrogen (H₂/CO) in syngas from CPO as a function of the conversion (%) and the molar ratio of hydrocarbons having four carbons (C₄) to carbon (C₄/C) in the reactant feed (in legend) at a pressure of 75 bar, an O₂ to carbon molar ratio (O₂/C) of 0.55, and without CO₂ in the reactant feed.

As seen in Figures 10 through 15, using reactant mixtures 5 comprising higher hydrocarbons (e.g., C₂, C₃, and/or C₄) allows a reduction in the amount of CO₂ utilized to reach a molar ratio of hydrogen to CO (H₂/CO) of about 1, and enables production of hydrogen-lean syngas having an H₂/CO molar ratio of about 1 at a higher hydrocarbon conversion to syngas.

The embodiments described herein are exemplary only, and are not intended to be limiting. Many variations and modifications of the subject matter disclosed herein are possible and are within the scope of the disclosure. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). For example, whenever a numerical range with a lower limit, RL and an upper limit, RU is disclosed, any number falling within the range is specifically disclosed. In particular, the following numbe1S within the range are specifically disclosed: R=R_{L}+k*(R_{U}-R_{L}),wherein k is a variable ranging from
1 percent to 100 percent with a 1 percent increment, i.e., k is 1 percent, 2 percent, 3 percent, 4 percent, 5 percent, ... 50 percent, 51 percent, 52 percent, . 95 percent, 96 percent, 97 percent, 98 percent, 99 percent, or 100 percent. Moreover, any numerical range defined by two R numbers as defined in the above is also specifically disclosed. Use of the term "optionally" with respect to any element of a claim is intended to mean that the subject element is required, or alternatively, is not required. Both alternatives are intended to be within the scope of the claim. Use of broader terms such as comprises, includes, having, etc. should be understood to provide support for narrower terms such as consisting of, consisting essentially of, comprised substantially of, etc.

Accordingly, the scope of protection is not limited by the description set out above but is only limited by the claims which follow. Each and every claim is incorporated into the specification as an embodiment of the present disclosure. Thus, the claims are a further description and are an addition to the embodiments of the present disclosure. The discussion of a reference is not an admission that it is prior alt to the present disclosure, especially any reference that may have a publication date after the priority date of this application. The disclosures of all patents, patent applications, and publications cited herein are hereby incorporated by reference, to the extent that they provide exemplary, procedural, or other details supplementary to those set forth herein.

### ADDITIONAL DESCRIPTION

The particular embodiments disclosed above are illustrative only, as the present disclosure may be modified and practiced in different but equivalent manners apparent to those skilled in the alt having the benefit of the teachings herein. Furthermore, no limitations are intended to the details of construction or design herein shown, other than as described in the claims below. Alternative embodiments that result from combining, integrating, and/or omitting features of the embodiment(s) are also within the scope of the disclosure. While compositions and methods are described in broader terms of "having", "comprising," "containing," or "including" various components or steps, the compositions and methods can also "consist essentially of' or "consist of the various components and steps. Use of the term "optionally" with respect to any element of a claim means that the element is required, or alternatively, the element is
not required, both alternatives being within the scope of the claim.

Numbers and ranges disclosed above may vary by some amount. Whenever a numerical range with a lower limit and an upper limit is disclosed, any number and any included range falling within the range are specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an", as used in the claims, are defined herein to mean one or more than one of the element that it introduces. If there is any conflict in the usages of a word or term in this specification and one or more patent or other documents, the definitions that are consistent with this specification should be adopted.

### Embodiments disclosed herein include:

A: A process for producing acetic acid according to claim 1[00**131**] B: A process for producing acetic acid according to claim 11.
C: A process for producing dimethyl ether (DME) according to claim 13.

Each of embodiments A, B, and C may have one or more of the following additional elements: Element 1: wherein the hydrocarbons comprise methane, natural gas, natural gas liquids, liquefied petroleum gas (LPG), associated gas, well head gas, enriched gas, paraffins, shale gas, shale liquids, fluid catalytic cracking (FCC) off gas, refinery process gases, refinery off gases, stack gases, fuel gas from a fuel gas header, or combinations thereof. Element 2: wherein the C₂₊ alkanes comprise ethane, propane, butanes, or combinations thereof. Element 3: wherein the CPO reactor is characterized by at least one CPO operational parameter selected from the group consisting of a CPO reactant mixture temperature of from 100 °C to 500 °C; a CPO pressure of from 20 barg to 80 barg; a CPO contact time of from 0.001 milliseconds (ms) to 5 seconds (s); a carbon to oxygen (C/O) molar ratio in the CPO reactant mixture of from 0.5:1 to 3: 1, wherein the C/O molar ratio refers to the total moles of carbon (C) in the hydrocarbons in the reactant mixture divided by the total moles of O₂ in the reactant mixture; a steam to carbon (SIC) molar ratio in the CPO reactant mixture of less than 0.6:1 , wherein the S/C molar ratio refelS to the total moles of water (H₂O) in the reactant mixture divided by the total moles of carbon (C) in the hydrocarbons in the reactant mixture; a CO₂ to carbon (CO₂/C) molar ratio in the CPO reactant mixture of equal to or greater than 0.5:1 , wherein the CO₂/C molar ratio refe1S to the total moles of CO₂ in the reactant mixture divided by the total moles of carbon (C) in the hydrocarbons in the reactant mixture; and combinations thereof. Element 4: excluding a step of introducing at least a portion of the H₂-leean syngas to a hydrogen recovery unit to decrease the amount of H₂ in the H₂-lean syngas. Element 5: wherein the CPO reactor is characterized by a CPO pressure; wherein the DME carbonylation unit is characterized by a DME carbonylation pressure; and wherein the CPO pressure is about the same as the DME carbonylation pressure. Element 6: wherein the CPO reactor is characterized by a CPO pressure; wherein the DME carbonylation unit is characterized by a DME carbonylation pressure; wherein at least a portion of the H₂-lean syngas is compressed in a compressor to yield a compressed syngas; wherein the compressed syngas is characterized by a pressure that is about the same as the DME carbonylation pressure; and wherein at least a portion of the compressed syngas is fed to the DME carbonylation unit in step (b). Element 7: wherein the compressor size is smaller than the size of a compressor used for compressing H₂-lean syngas
in an otherwise similar process that (i) employs a H₂ recovery unit for producing a H₂-lean syngas and/or (ii) produces a H₂-lean syngas from hydrocarbons comprising less than 3 mol C₂₊ alkanes. Element 8: further comprising feeding at least a portion of the H₂-enriched syngas to a methanol synthesis unit to produce another methanol stream and a purge gas stream, wherein the purge gas stream comprises H₂, CO, CO₂, and unreacted hydrocarbons, and wherein at least a portion of the purge gas stream is optionally used as fuel. Element 9: further comprising: (1) feeding at least a portion of the methanol stream and/or at least a portion of the another methanol stream to a DME synthesis reaction zone to produce a DME stream; and (2) feeding at least a portion of the DME stream to the DME carbonylation unit in step (b). Element 10: wherein a common reactor comprises both the methyl acetate hydrolysis reaction zone and the DME synthesis reaction zone. Element 1 1: wherein the amount of acetic acid produced is greater than the amount of acetic acid produced in an otherwise similar process that (i) employs a H₂ recovery unit for producing a H₂-lean syngas and/or (ii) produces a H₂-lean syngas from hydrocarbons comprising less than 3 mol C₂₊ alkanes. Element 12: wherein the CPO reactor is characterized by at least one CPO operational parameter selected from the group consisting of a CPO reactant mixture temperature of from 100 °C to 500 °C; a CPO pressure of from 25 barg to 80 barg; a CPO contact time of from 0.001 milliseconds (ms) to 5 seconds (s); a C/O molar ratio in the CPO reactant mixture of from 0.5:1 to 2:1, wherein the C/O molar ratio refelS to the total moles of carbon (C) in the hydrocarbons in the reactant mixture divided by the total moles of O₂ in the reactant mixture; a S/C molar ratio in the CPO reactant mixture of less than 0.25:1 , wherein the S/C molar ratio refe1S to the total moles of water in the reactant mixture divided by the total moles of carbon (C) in the hydrocarbons in the reactant mixture; a CO₂/C molar ratio in the CPO reactant mixture of equal to or greater than 0.5 : 1 , wherein the CO₂/C molar ratio refers to the total moles of CO₂ in the reactant mixture divided by the total moles of carbon (C) in the hydrocarbons in the reactant mixture; and combinations thereof. Element 13: further comprising: (1) optionally compressing at least a portion of H₂-lean syngas to yield a compressed syngas, wherein the pressure of the compressed syngas is about the same as the DME reactor pressure; and (2) feeding at least a portion of the compressed syngas to DME reactor in step (b). Element 14: wherein the amount of DME produced is greater than the amount of DME produced in an otherwise similar process that (i) employs a H₂ recovery unit for producing a H₂-lean syngas and/or (ii) produces a H₂-lean syngas from hydrocarbons comprising less than 3 mol% C₂₊ alkanes.

While preferred embodiments of the invention have been shown and described, modifications thereof can be made by one skilled in the art without departing from the teachings of this disclosure. The embodiments described herein are exemplary only, and are not intended to be limiting. Many variations and modifications of the invention disclosed herein are possible and are within the scope of the invention.

Numerous other modifications, equivalents, and alternatives, will become apparent to those skilled in the alt once the above disclosure is fully appreciated. Accordingly, the scope of protection is not limited by the description set out above but is only limited by the claims which follow. Each and every claim is incorporated into the specification as an embodiment of the present invention. Thus, the claims are a further description and are an addition to the detailed description of the present invention.

## Claims

1. A process for producing acetic acid comprising:
(a) reacting, via a catalytic partial oxidation (CPO) reaction, a CPO reactant mixture in a CPO reactor to produce a hydrogen-lean syngas; wherein the CPO reactant mixture comprises hydrocarbons, oxygen, carbon dioxide and optionally steam; wherein the hydrocarbons comprise equal to or greater than 3 mol% C₂₊alkanes; wherein the CPO reactor comprises a CPO catalyst; wherein the hydrogen-lean syngas comprises hydrogen, carbon monoxide, carbon dioxide, and unreacted hydrocarbons; and wherein the hydrogen-lean syngas is **characterized by** a hydrogen to carbon monoxide (H₂/CO) molar ratio of from 0.7 to 1.3;
(b) feeding at least a portion of the hydrogen-lean syngas and dimethyl ether (DME) to a DME carbonylation unit to produce methyl acetate and a hydrogen-enriched syngas; wherein the hydrogen-enriched syngas comprises hydrogen, carbon monoxide, carbon dioxide, and unreacted hydrocarbons, and wherein the hydrogen-enriched syngas is **characterized by** a H₂/CO molar ratio of from 1.4 to 2.2; and
(c) feeding at least a portion of the methyl acetate and water to a methyl acetate hydrolysis reaction zone to produce acetic acid and a methanol stream.

2. The process of claim 1, wherein the hydrocarbons comprise methane, natural gas, natural gas liquids, liquefied petroleum gas (LPG), associated gas, well head gas, enriched gas, paraffins, shale gas, shale liquids, fluid catalytic cracking (FCC) off gas, refinery process gases, refinery off gases, stack gases, fuel gas from a fuel gas header, or combinations thereof.

3. The process of any of claims 1-2, wherein the C₂₊alkanes comprise ethane, propane, butanes, or combinations thereof.

4. The process of any of claims 1-3, wherein the CPO reactor is **characterized by** at least one CPO operational parameter selected from the group consisting of a CPO reactant mixture temperature of from 100 °C to 500 °C; a CPO pressure of from 20 barg to 80 barg; a CPO contact time of from 0.001 milliseconds (ms) to t 5 seconds (s); a carbon to oxygen (C/O) molar ratio in the CPO reactant mixture of from 0.5 : 1 to 3:1, wherein the C/O molar ratio refers to the total moles of carbon (C) in the hydrocarbons in the reactant mixture divided by the total moles of oxygen (O₂) in the reactant mixture; a steam to carbon (S/C) molar ratio in the CPO reactant mixture of less than 0.6:1, wherein the S/C molar ratio refers to the total moles of water (H₂O) in the reactant mixture divided by the total moles of carbon (C) in the hydrocarbons in the reactant mixture; a CO₂ to carbon (CO₂/C) molar ratio in the CPO reactant mixture of equal to or greater than 0.5:1, wherein the CO₂/C molar ratio refers to the total moles of CO₂ in the reactant mixture divided by the total moles of carbon (C) in the hydrocarbons in the reactant mixture; and combinations thereof.

5. The process of any of claims 1-4 excluding a step of introducing at least a portion of the hydrogen-lean syngas to a hydrogen recovery unit to decrease the amount of hydrogen in the hydrogen-lean syngas.

6. The process of any of claims 1-5, wherein the CPO reactor is **characterized by** a CPO pressure; wherein the DME carbonylation unit is **characterized by** a DME carbonylation pressure; and wherein the CPO pressure is about the same as the DME carbonylation pressure.

7. The process of any of claims 1-6, wherein the CPO reactor is **characterized by** a CPO pressure; wherein the DME carbonylation unit is **characterized by** a DME carbonylation pressure; wherein at least a portion of the hydrogen-lean syngas is compressed in a compressor to yield a compressed syngas; wherein the compressed syngas is **characterized by** a pressure that is about the same as the DME carbonylation pressure; and wherein at least a portion of the compressed syngas is fed to the DME carbonylation unit in step (b).

8. The process of any of claims 1-7 further comprising feeding at least a portion of the hydrogen-enriched syngas to a methanol synthesis unit to produce another methanol stream and a purge gas stream, wherein the purge gas stream comprises hydrogen, carbon monoxide, carbon dioxide, and unreacted hydrocarbons, and wherein at least a portion of the purge gas stream is optionally used as fuel.

9. The process of claim 8 further comprising: ( 1 ) feeding at least a portion of the methanol stream and/or at least a portion of the another methanol stream to a DME synthesis reaction zone to produce a DME stream; and (2) feeding at least a portion of the DME stream to the DME carbonylation unit in step (b).

10. The process of claim 9, wherein a common reactor comprises both the methyl acetate hydrolysis reaction zone and the DME synthesis reaction zone.

11. A process for producing acetic acid according to claim 1 comprising:
(a) reacting, via a catalytic partial oxidation (CPO) reaction, a CPO reactant mixture in a CPO reactor to produce a hydrogen-lean syngas; wherein the CPO reactant mixture comprises hydrocarbons, oxygen, carbon dioxide and optionally steam; wherein the hydrocarbons comprise equal to or greater than 5 mol% C₂₊ alkanes; wherein the CPO reactor comprises a CPO catalyst; wherein the CPO reactor is **characterized by** a CPO pressure; wherein the hydrogen-lean syngas comprises hydrogen, carbon monoxide, carbon dioxide, and unreacted hydrocarbons; and wherein the hydrogen-lean syngas is **characterized by** a hydrogen to carbon monoxide (H₂/CO) molar ratio of from 0.8 to 1.3;
(b) optionally compressing at least a portion of the hydrogen-lean syngas to yield a compressed syngas;
(c) feeding at least a portion of the hydrogen-lean syngas and/or compressed syngas, and dimethyl ether (DME) to a DME carbonylation unit to produce methyl acetate and a hydrogen-enriched syngas; wherein the DME carbonylation unit is **characterized by** a DME carbonylation pressure; wherein the pressure of the hydrogen-lean syngas and/or compressed syngas is about the same as the DME carbonylation pressure; wherein the hydrogen-enriched syngas comprises hydrogen, carbon monoxide, carbon dioxide, and unreacted hydrocarbons, and wherein the hydrogen-enriched syngas is **characterized by** a H₂/CO molar ratio of from 1.4 to 2.2;
(d) feeding at least a portion of the methyl acetate and water to a methyl acetate hydrolysis reaction zone to produce acetic acid and a first methanol stream;
(e) feeding at least a portion of the hydrogen-enriched syngas to a methanol synthesis unit to produce a second methanol stream and a purge gas stream, wherein the purge gas stream comprises hydrogen, carbon monoxide, carbon dioxide, and unreacted hydrocarbons;
(f) feeding at least a portion of the first methanol stream and/or at least a portion of the second methanol stream to a DME synthesis reaction zone to produce a DME stream, wherein a common reactor comprises both the methyl acetate hydrolysis reaction zone and the DME synthesis reaction zone; and
(g) feeding at least a portion of the DME stream to the DME carbonylation unit in step (c).

12. The process of claim 11, wherein the CPO reactor is **characterized by** at least one CPO operational parameter selected from the group consisting of a CPO reactant mixture temperature of from 100 °C to 500 °C; a CPO pressure of from 25 barg to 80 barg; a CPO contact time of from 0.001 milliseconds (ms) to 5 seconds (s); a carbon to oxygen (C/O) molar ratio in the CPO reactant mixture of from 0.5:1 to 2:1, wherein the C/O molar ratio refers to the total moles of carbon (C) in the hydrocarbons in the reactant mixture divided by the total moles of oxygen (O₂) in the reactant mixture; a steam to carbon (S/C) molar ratio in the CPO reactant mixture of less than 0.25:1, wherein the S/C molar ratio refers to the total moles of water (H₂O) in the reactant mixture divided by the total moles of carbon (C) in the hydrocarbons in the reactant mixture; a CO₂ to carbon (CO₂/C) molar ratio in the CPO reactant mixture of equal to or greater than 0.5:1, wherein the CO₂/C molar ratio refers to the total moles of CO₂ in the reactant mixture divided by the total moles of carbon (C) in the hydrocarbons in the reactant mixture; and combinations thereof.

13. A process for producing dimethyl ether (DME) comprising:
(a) reacting, via a catalytic partial oxidation (CPO) reaction, a CPO reactant mixture in a CPO reactor to produce a hydrogen-lean syngas; wherein the CPO reactant mixture comprises hydrocarbons, oxygen, carbon dioxide and optionally steam; wherein the hydrocarbons comprise equal to or greater than 3 mol% C₂₊ alkanes; wherein the CPO reactor comprises a CPO catalyst; wherein the CPO reactor is **characterized by** a CPO pressure; wherein the hydrogen-lean syngas comprises hydrogen, carbon monoxide, carbon dioxide, and unreacted hydrocarbons; and wherein the hydrogen-lean syngas is **characterized by** a hydrogen to carbon monoxide (H₂/CO) molar ratio of from 0.8 to 1.3;
(b) feeding at least a portion of the hydrogen-lean syngas to a dimethyl ether (DME) reactor to produce a DME reactor effluent; wherein the DME reactor is **characterized by** a DME reactor pressure; wherein the CPO pressure and the DME reactor pressure are the same or different; wherein the DME reactor effluent comprises DME, methanol, water, and carbon dioxide;
(c) separating at least a portion of the DME reactor effluent into a DME stream, a methanol stream, a water stream, and a carbon dioxide stream;
(d) optionally recycling at least a portion of the methanol stream to the DME reactor, and
(e) optionally recycling at least a portion of the carbon dioxide stream to the CPO reactor.

14. The process of claim 13, wherein the hydrocarbons comprise methane, natural gas, natural gas liquids, liquefied petroleum gas (LPG), associated gas, well head gas, enriched gas, paraffins, shale gas, shale liquids, fluid catalytic cracking (FCC) off gas, refinery process gases, refinery off gases, stack gases, fuel gas from a fuel gas header, or combinations thereof, and wherein the C₂₊ alkanes comprise ethane, propane, butanes, or combinations thereof.

15. The process of any of claims 13-14, wherein the CPO reactor is **characterized by** at least one CPO operational parameter selected from the group consisting of a CPO reactant mixture temperature of from 100 °C to 500 °C; a CPO pressure of from 20 barg to 80 barg; a CPO contact time of from 0.001 milliseconds (ms) to 5 seconds (s); a carbon to oxygen (C/O) molar ratio in the CPO reactant mixture of from 0.5:1 to 3:1, wherein the C/O molar ratio refers to the total moles of carbon (C) in the hydrocarbons in the reactant mixture divided by the total moles of oxygen (O₂) in the reactant mixture; a steam to carbon (S/C) molar ratio in the CPO reactant mixture of less than 0.6:1, wherein the S/C molar ratio refers to the total moles of water (H₂O) in the reactant mixture divided by the total moles of carbon (C) in the hydrocarbons in the reactant mixture; a CO₂ to carbon (CO₂/C) molar ratio in the CPO reactant mixture of equal to or greater than 0.5:1, wherein the CO₂/C molar ratio refers to the total moles of CO₂ in the reactant mixture divided by the total moles of carbon (C) in the hydrocarbons in the reactant mixture; and combinations thereof.

16. The process of any of claims 13-15 excluding a step of introducing at least a portion of the hydrogen-lean syngas to a hydrogen recovery unit to decrease the amount of hydrogen in the hydrogen-lean syngas.

17. The process of any of claims 13-16 further comprising: (1) optionally compressing at least a portion of the hydrogen-lean syngas to yield a compressed syngas, wherein the pressure of the compressed syngas is about the same as the DME reactor pressure; and (2) feeding at least a portion of the compressed syngas to the DME reactor in step (b).

## Patentansprüche

1. Verfahren zur Herstellung von Essigsäure, umfassend:
(a) Umsetzen, mittels einer katalytischen Teiloxidationsreaktion (CPO), einer CPO-Reaktantenmischung in einem CPO-Reaktor, um ein wasserstoffarmes Synthesegas zu erzeugen; wobei die CPO-Reaktantenmischung Kohlenwasserstoffe, Sauerstoff, Kohlendioxid und optional Wasserdampf umfasst; wobei die Kohlenwasserstoffe 3 Mol-% C₂₊Alkane oder mehr umfassen; wobei der CPO-Reaktor einen CPO-Katalysator umfasst; wobei das wasserstoffarme Synthesegas Wasserstoff, Kohlenmonoxid, Kohlendioxid und nicht reagierte Kohlenwasserstoffe umfasst; und wobei das wasserstoffarme Synthesegas durch ein Molverhältnis von Wasserstoff zu Kohlenmonoxid (H₂/CO) von 0,7 bis 1,3 gekennzeichnet ist;
(b) Zuführen mindestens eines Teils des wasserstoffarmen Synthesegases und Dimethylether (DME) zu einer DME-Carbonylierungseinheit, um Methylacetat und ein wasserstoffangereichertes Synthesegas zu erzeugen; wobei das wasserstoffangereicherte Synthesegas Wasserstoff, Kohlenmonoxid, Kohlendioxid und nicht reagierte Kohlenwasserstoffe umfasst, und wobei das wasserstoffangereicherte Synthesegas durch ein H₂/CO-Molverhältnis von 1,4 bis 2,2 gekennzeichnet ist; und
(c) Zuführen mindestens eines Teils des Methylacetats und Wasser zu einer Methylacetat-Hydrolyse-Reaktionszone, um Essigsäure und einen Methanolstrom zu erzeugen.

2. Verfahren nach Anspruch 1, wobei die Kohlenwasserstoffe Methan, Erdgas, Erdgasflüssigkeiten, Flüssiggas (LPG), Begleitgas, Bohrlochkopfgas, angereichertes Gas, Paraffine, Schiefergas, Schieferflüssigkeiten, Abgas aus dem Fluid Catalytic Cracking (FCC), Raffinerieprozessgase, Raffinerieabgase, Kaminabgase, Brenngas aus einem Brenngasverteiler oder Kombinationen davon umfassen.

3. Verfahren nach Anspruch 1 oder 2, wobei die C₂₊-Alkane Ethan, Propan, Butane oder Kombinationen davon umfassen.

4. Verfahren nach einem der Ansprüche 1 bis 3, wobei der CPO-Reaktor durch mindestens einen CPO-Betriebsparameter gekennzeichnet ist, ausgewählt aus der Gruppe bestehend aus einer Temperatur der CPO-Reaktantenmischung von 100 °C bis 500 °C; einen CPO-Druck von 20 barg bis 80 barg; eine CPO-Verweilzeit von 0,001 Millisekunden (ms) bis 15 Sekunden (s); einem Molverhältnis von Kohlenstoff zu Sauerstoff (C/O) in der CPO-Reaktantenmischung von 0,5:1 bis 3:1, wobei sich das C/O-Molverhältnis auf die Gesamtmolzahl an Kohlenstoff (C) in den Kohlenwasserstoffen der Reaktantenmischung geteilt durch die Gesamtmolzahl an Sauerstoff (O₂) in der Reaktantenmischung bezieht; ein Molverhältnis von Wasserdampf zu Kohlenstoff (S/C) in der CPO-Reaktantenmischung von weniger als 0,6:1, wobei sich das S/C-Molverhältnis auf die Gesamtmolzahl von Wasser (H₂O) in der Reaktantenmischung geteilt durch die Gesamtmolzahl von Kohlenstoff (C) in den Kohlenwasserstoffen in der Reaktantenmischung bezieht; ein Molverhältnis von CO₂ zu Kohlenstoff (CO₂/C) in der CPO-Reaktantenmischung von 0,5:1 oder mehr, wobei sich das CO₂/C-Molverhältnis auf die Gesamtmolzahl an CO₂ in der Reaktantenmischung geteilt durch die Gesamtmolzahl an Kohlenstoff (C) in den Kohlenwasserstoffen der Reaktantenmischung bezieht; und Kombinationen davon.

5. Verfahren nach einem der Ansprüche 1 bis 4, ausgenommen einen Schritt des Einleitens mindestens eines Teils des wasserstoffarmen Synthesegases in eine Wasserstoffrückgewinnungseinheit, um die Wasserstoffmenge im wasserstoffarmen Synthesegas zu verringern.

6. Verfahren nach einem der Ansprüche 1 bis 5, wobei der CPO-Reaktor durch einen CPO-Druck gekennzeichnet ist; wobei die DME-Carbonylierungseinheit durch einen DME-Carbonylierungsdruck gekennzeichnet ist; und wobei der CPO-Druck in etwa dem DME-Carbonylierungsdruck entspricht.

7. Verfahren nach einem der Ansprüche 1 bis 6, wobei der CPO-Reaktor durch einen CPO-Druck gekennzeichnet ist; wobei die DME-Carbonylierungseinheit durch einen DME-Carbonylierungsdruck gekennzeichnet ist; wobei mindestens ein Teil des wasserstoffarmen Synthesegases in einem Kompressor komprimiert wird, um ein komprimiertes Synthesegas zu erzeugen; wobei das komprimierte Synthesegas durch einen Druck gekennzeichnet ist, der in etwa dem DME-Carbonylierungsdruck entspricht; und wobei mindestens ein Teil des komprimierten Synthesegases der DME-Carbonylierungseinheit in Schritt (b) zugeführt wird.

8. Verfahren nach einem der Ansprüche 1 bis 7, das ferner das Zuführen mindestens eines Teils des wasserstoffangereicherten Synthesegases zu einer Methanolsyntheseeinheit umfasst, um einen weiteren Methanolstrom und einen Spülgasstrom zu erzeugen, wobei der Spülgasstrom Wasserstoff, Kohlenmonoxid, Kohlendioxid und nicht reagierte Kohlenwasserstoffe umfasst und wobei mindestens ein Teil des Spülgasstroms optional als Brennstoff verwendet wird.

9. Verfahren nach Anspruch 8, ferner umfassend: (1) Zuführen mindestens eines Teils des Methanolstroms und/oder mindestens eines Teils des weiteren Methanolstroms zu einer DME-Synthesereaktionszone, um einen DME-Strom zu erzeugen; und (2) Zuführen mindestens eines Teils des DME-Stroms zur DME-Carbonylierungseinheit in Schritt (b).

10. Verfahren nach Anspruch 9, wobei ein gemeinsamer Reaktor sowohl die Methylacetat-Hydrolysereaktionszone als auch die DME-Synthesereaktionszone umfasst.

11. Verfahren zur Herstellung von Essigsäure nach Anspruch 1, umfassend:
(a) Umsetzen, mittels einer katalytischen Teiloxidationsreaktion (CPO), einer CPO-Reaktantenmischung in einem CPO-Reaktor, um ein wasserstoffarmes Synthesegas zu erzeugen; wobei die CPO-Reaktantenmischung Kohlenwasserstoffe, Sauerstoff, Kohlendioxid und optional Wasserdampf umfasst; wobei die Kohlenwasserstoffe 5 Mol-% C₂₊Alkane oder mehr umfassen; wobei der CPO-Reaktor einen CPO-Katalysator umfasst; wobei der CPO-Reaktor durch einen CPO-Druck gekennzeichnet ist; wobei das wasserstoffarme Synthesegas Wasserstoff, Kohlenmonoxid, Kohlendioxid und nicht reagierte Kohlenwasserstoffe umfasst; und wobei das wasserstoffarme Synthesegas durch ein Molverhältnis von Wasserstoff zu Kohlenmonoxid (H₂/CO) von 0,8 bis 1,3 gekennzeichnet ist;
(b) optional Komprimieren mindestens eines Teils des wasserstoffarmen Synthesegases, um ein komprimiertes Synthesegas zu erhalten;
(c) Zuführen mindestens eines Teils des wasserstoffarmen Synthesegases und/oder des komprimierten Synthesegases sowie Dimethylether (DME) zu einer DME-Carbonylierungseinheit, um Methylacetat und ein wasserstoffangereichertes Synthesegas zu erzeugen; wobei die DME-Carbonylierungseinheit durch einen DME-Carbonylierungsdruck gekennzeichnet ist; wobei der Druck des wasserstoffarmen Synthesegases und/oder des komprimierten Synthesegases in etwa dem DME-Carbonylierungsdruck entspricht; wobei das wasserstoffangereicherte Synthesegas Wasserstoff, Kohlenmonoxid, Kohlendioxid und nicht reagierte Kohlenwasserstoffe umfasst und wobei das wasserstoffangereicherte Synthesegas durch ein H₂/CO-Molverhältnis von 1,4 bis 2,2 gekennzeichnet ist;
(d) Zuführen mindestens eines Teils des Methylacetats und des Wassers zu einer Methylacetat-Hydrolysereaktionszone, um Essigsäure und einen ersten Methanolstrom zu erzeugen;
(e) Zuführen mindestens eines Teils des wasserstoffangereicherten Synthesegases zu einer Methanolsyntheseanlage, um einen zweiten Methanolstrom und einen Spülgasstrom zu erzeugen, wobei der Spülgasstrom Wasserstoff, Kohlenmonoxid, Kohlendioxid und nicht reagierte Kohlenwasserstoffe umfasst;
(f) Zuführen mindestens eines Teils des ersten Methanolstroms und/oder mindestens eines Teils des zweiten Methanolstroms zu einer DME-Synthesereaktionszone, um einen DME-Strom zu erzeugen, wobei ein gemeinsamer Reaktor sowohl die Methylacetat-Hydrolysereaktionszone als auch die DME-Synthesereaktionszone umfasst; und
(g) Zuführen mindestens eines Teils des DME-Stroms zur DME-Carbonylierungseinheit in Schritt (c).

12. Verfahren nach Anspruch 11, wobei der CPO-Reaktor durch mindestens einen CPO-Betriebsparameter gekennzeichnet ist, ausgewählt aus der Gruppe bestehend aus einer Temperatur der CPO-Reaktantenmischung von 100 °C bis 500 °C; einem CPO-Druck von 25 barg bis 80 barg; einer CPO-Verweilzeit von 0,001 Millisekunden (ms) bis 5 Sekunden (s); einem Molverhältnis von Kohlenstoff zu Sauerstoff (C/O) in der CPO-Reaktantenmischung von 0,5:1 bis 2:1, wobei sich das C/O-Molverhältnis auf die Gesamtmolzahl an Kohlenstoff (C) in den Kohlenwasserstoffen der Reaktantenmischung geteilt durch die Gesamtmolzahl an Sauerstoff (O₂) in der Reaktantenmischung bezieht; ein Molverhältnis von Wasserdampf zu Kohlenstoff (S/C) in der CPO-Reaktantenmischung von weniger als 0,25:1, wobei sich das S/C-Molverhältnis auf die Gesamtmolzahl von Wasser (H₂O) in der Reaktantenmischung geteilt durch die Gesamtmolzahl von Kohlenstoff (C) in den Kohlenwasserstoffen der Reaktantenmischung bezieht; ein Molverhältnis von CO₂ zu Kohlenstoff (CO₂/C) in der CPO-Reaktantenmischung von 0,5:1 oder mehr, wobei sich das CO₂/C-Molverhältnis auf die Gesamtmolzahl an CO₂ in der Reaktantenmischung geteilt durch die Gesamtmolzahl an Kohlenstoff (C) in den Kohlenwasserstoffen der Reaktantenmischung bezieht; und Kombinationen davon.

13. Verfahren zur Herstellung von Dimethylether (DME), umfassend:
(a) Umsetzen, mittels einer katalytischen Teiloxidationsreaktion (CPO), einer CPO-Reaktantenmischung in einem CPO-Reaktor, um ein wasserstoffarmes Synthesegas zu erzeugen; wobei die CPO-Reaktantenmischung Kohlenwasserstoffe, Sauerstoff, Kohlendioxid und optional Wasserdampf umfasst; wobei die Kohlenwasserstoffe 3 Mol-% C₂₊-Alkane oder mehr umfassen;
wobei der CPO-Reaktor einen CPO-Katalysator umfasst; wobei der CPO-Reaktor durch einen CPO-Druck gekennzeichnet ist; wobei das wasserstoffarme Synthesegas Wasserstoff, Kohlenmonoxid, Kohlendioxid und nicht reagierte Kohlenwasserstoffe umfasst; und wobei das wasserstoffarme Synthesegas durch ein Molverhältnis von Wasserstoff zu Kohlenmonoxid (H₂/CO) von 0,8 bis 1,3 gekennzeichnet ist;
(b) Zuführen mindestens eines Teils des wasserstoffarmen Synthesegases zu einem Dimethylether (DME)-Reaktor, um ein DME-Reaktorabgas zu erzeugen; wobei der DME-Reaktor durch einen DME-Reaktordruck gekennzeichnet ist; wobei der CPO-Druck und der DME-Reaktordruck gleich oder unterschiedlich sind; wobei das DME-Reaktorabgas DME, Methanol, Wasser und Kohlendioxid umfasst;
(c) Trennen mindestens eines Teils des DME-Reaktorabflusses in einen DME-Strom, einen Methanolstrom, einen Wasserstrom und einen Kohlendioxidstrom;
(d) optional Rückführen mindestens eines Teils des Methanolstroms in den DME-Reaktor, und
(e) optional Rückführen mindestens eines Teils des Kohlendioxidstroms in den CPO-Reaktor.

14. Verfahren nach Anspruch 13, wobei die Kohlenwasserstoffe Methan, Erdgas, Erdgasflüssigkeiten, Flüssiggas (LPG), Begleitgas, Bohrlochkopfgas, angereichertes Gas, Paraffine, Schiefergas, Schieferflüssigkeiten, Abgas aus dem Fluid Catalytic Cracking (FCC), Raffinerieprozessgase, Raffinerieabgase, Kaminabgase, Brenngas aus einem Brenngasverteiler oder Kombinationen davon umfassen, und wobei die C₂₊-Alkane Ethan, Propan, Butane oder Kombinationen davon umfassen.

15. Verfahren nach einem der Ansprüche 13 bis 14, wobei der CPO-Reaktor durch mindestens einen CPO-Betriebsparameter gekennzeichnet ist, ausgewählt aus der Gruppe bestehend aus einer Temperatur der CPO-Reaktantenmischung von 100 °C bis 500 °C; einem CPO-Druck von 20 barg bis 80 barg; einer CPO-Verweilzeit von 0,001 Millisekunden (ms) bis 5 Sekunden (s); einem Molverhältnis von Kohlenstoff zu Sauerstoff (C/O) in der CPO-Reaktantenmischung von 0,5:1 bis 3:1, wobei sich das C/O-Molverhältnis auf die Gesamtmolzahl an Kohlenstoff (C) in den Kohlenwasserstoffen der Reaktantenmischung geteilt durch die Gesamtmolzahl an Sauerstoff (O₂) in der Reaktantenmischung bezieht; ein Molverhältnis von Wasserdampf zu Kohlenstoff (S/C) in der CPO-Reaktantenmischung von weniger als 0,6:1, wobei sich das S/C-Molverhältnis auf die Gesamtmolzahl von Wasser (H₂O) in der Reaktantenmischung geteilt durch die Gesamtmolzahl von Kohlenstoff (C) in den Kohlenwasserstoffen in der Reaktantenmischung bezieht; ein Molverhältnis von CO₂ zu Kohlenstoff (CO₂/C) in der CPO-Reaktantenmischung 0,5:1 oder mehr, wobei sich das CO₂/C-Molverhältnis auf die Gesamtmolzahl an CO₂ in der Reaktantenmischung geteilt durch die Gesamtmolzahl an Kohlenstoff (C) in den Kohlenwasserstoffen in der Reaktantenmischung bezieht; und Kombinationen davon.

16. Verfahren nach einem der Ansprüche 13 bis 15, ausgenommen einen Schritt des Einleitens mindestens eines Teils des wasserstoffarmen Synthesegases in eine Wasserstoffrückgewinnungseinheit, um die Wasserstoffmenge im wasserstoffarmen Synthesegas zu verringern.

17. Verfahren nach einem der Ansprüche 13 bis 16, ferner umfassend: (1) optional Komprimieren mindestens eines Teils des wasserstoffarmen Synthesegases, um ein komprimiertes Synthesegas zu erhalten, wobei der Druck des komprimierten Synthesegases in etwa dem DME-Reaktordruck entspricht; und (2) Zuführen mindestens eines Teils des komprimierten Synthesegases zum DME-Reaktor in Schritt (b).

## Revendications

1. Procédé de production d'acide acétique, comprenant les étapes consistant à:
(a) faire réagir, par une réaction d'oxydation partielle catalytique (CPO), un mélange de partenaires réactionnels de CPO dans un réacteur de CPO pour produire un gaz de synthèse à faible teneur en hydrogène; le mélange de partenaires réactionnels de CPO comprenant des hydrocarbures, de l'oxygène, du dioxyde de carbone et en option de la vapeur d'eau; les hydrocarbures comprenant au moins 3 % en moles d'alcanes en C₂₊; le réacteur de CPO comprenant un catalyseur de CPO; le gaz de synthèse à faible teneur en hydrogène comprenant de l'hydrogène, du monoxyde de carbone, du dioxyde de carbone et des hydrocarbures n'ayant pas réagi; et le gaz de synthèse à faible teneur en hydrogène étant **caractérisé par** un rapport molaire d'hydrogène à monoxyde de carbone (H₂/CO) valant de 0,7 à 1,3;
(b) introduire au moins une partie du gaz de synthèse à faible teneur en hydrogène et de l'éther diméthylique (DME) dans une unité de carbonylation de DME pour produire de l'acétate de méthyle et un gaz de synthèse enrichi en hydrogène; le gaz de synthèse enrichi en hydrogène comprenant de l'hydrogène, du monoxyde de carbone, du dioxyde de carbone, et des hydrocarbures n'ayant pas réagi, et le gaz de synthèse enrichi en hydrogène étant **caractérisé par** un rapport molaire H₂/CO valant de 1,4 à 2,2; et
(c) introduire au moins une partie de l'acétate de méthyle et de l'eau dans une zone de réaction d'hydrolyse d'acétate de méthyle pour produire de l'acide acétique et un courant de méthanol.

2. Procédé selon la revendication 1, dans lequel les hydrocarbures comprennent le méthane, le gaz naturel, les liquides de gaz naturel, le gaz de pétrole liquéfié (GPL), le gaz associé, le gaz de tête de puits, le gaz enrichi, les paraffines, le gaz de schiste, les liquides de schiste, le gaz résiduel de craquage catalytique fluide (FCC), les gaz de processus de raffinerie, les gaz résiduels de raffinerie, les gaz de combustion, le gaz combustible provenant d'un collecteur de gaz combustible, ou des associations de de ceux-ci.

3. Procédé selon l'une quelconque des revendications 1 et 2, dans lequel les alcanes en C₂₊ comprennent l'éthane, le propane, les butanes, ou des associations de ceux-ci.

4. Procédé selon l'une quelconque des revendications 1-3, dans lequel le réacteur de CPO est **caractérisé par** au moins un paramètre opérationnel de CPO choisi dans l'ensemble constitué par une température du mélange de partenaires réactionnels de CPO valant de 100 °C à 500 °C; une pression manométrique de CPO valant de 20 bar à 80 bar; un temps de contact de CPO valant de 0,001 milliseconde (ms) à 5 secondes (s); un rapport molaire de carbone à oxygène (C/O) dans le mélange de partenaires réactionnels de CPO valant de 0,5:1 à 3:1, le rapport molaire C/O désignant le nombre total de moles de carbone (C) dans les hydrocarbures dans le mélange de partenaires réactionnels divisé par le nombre total de moles d'oxygène (O₂) dans le mélange de partenaires réactionnels; un rapport molaire de vapeur d'eau à carbone (V/C) dans le mélange de partenaires réactionnels de CPO inférieur à 0,6:1, le rapport molaire V/C désignant le nombre total de moles d'eau (H₂O) dans le mélange de partenaires réactionnels divisé par le nombre total de moles de carbone (C) dans les hydrocarbures dans le mélange de partenaires réactionnels; un rapport molaire de CO₂ à carbone (CO₂/C) dans le mélange de partenaires réactionnels de CPO égal ou supérieur à 0,5:1, le rapport molaire CO₂/C désignant le nombre total de moles de CO₂ dans le mélange de partenaires réactionnels divisé par le nombre total de moles de carbone (C) dans les hydrocarbures dans le mélange de partenaires réactionnels; et des associations de tels paramètres.

5. Procédé selon l'une quelconque des revendications 1-4, excluant une étape consistant à introduire au moins une partie du gaz de synthèse à faible teneur en hydrogène dans une unité de récupération d'hydrogène pour diminuer la quantité d'hydrogène dans le gaz de synthèse à faible teneur en hydrogène.

6. Procédé selon l'une quelconque des revendications 1-5, dans lequel le réacteur de CPO est **caractérisé par** une pression de CPO; dans lequel l'unité de carbonylation de DME est **caractérisée par** une pression de carbonylation de DME; et dans lequel la pression de CPO est approximativement identique à la pression de carbonylation de DME.

7. Procédé selon l'une quelconque des revendications 1-6, dans lequel le réacteur de CPO est **caractérisé par** une pression de CPO; dans lequel l'unité de carbonylation de DME est **caractérisée par** une pression de carbonylation de DME; dans lequel au moins une partie du gaz de synthèse à faible teneur en hydrogène est comprimée dans un compresseur pour produire un gaz de synthèse comprimé; dans lequel le gaz de synthèse comprimé est **caractérisé par** une pression qui est approximativement identique à la pression de carbonylation de DME; et dans lequel au moins une partie du gaz de synthèse comprimé est introduite dans l'unité de carbonylation de DME dans l'étape (b).

8. Procédé selon l'une quelconque des revendications 1-7, comprenant en outre introduire dans une unité de synthèse de méthanol au moins une partie du gaz de synthèse enrichi en hydrogène pour produire un autre courant de méthanol et un courant de gaz de purge, le courant de gaz de purge comprenant de l'hydrogène, du monoxyde de carbone, du dioxyde de carbone, et des hydrocarbures n'ayant pas réagi, et dans lequel au moins une partie du courant de gaz de purge est en option utilisée comme combustible.

9. Procédé selon la revendication 8, comprenant en outre: (1) introduire au moins une partie du courant de méthanol et/ou au moins une partie de l'autre courant de méthanol dans une zone de réaction de synthèse de DME pour produire un courant de DME; et (2) introduire au moins une partie du courant de DME dans l'unité de carbonylation de DME dans l'étape (b).

10. Procédé selon la revendication 9, dans lequel un réacteur commun comprend à la fois la zone de réaction d'hydrolyse d'acétate de méthyle et la zone de réaction de synthèse de DME.

11. Procédé de production d'acide acétique selon la revendication 1, comprenant les étapes consistant à :
(a) faire réagir, par une réaction d'oxydation partielle catalytique (CPO), un mélange de partenaires réactionnels de CPO dans un réacteur de CPO pour produire un gaz de synthèse à faible teneur en hydrogène; le mélange de partenaires réactionnels de CPO comprenant des hydrocarbures, de l'oxygène, du dioxyde de carbone et en option de la vapeur d'eau; les hydrocarbures comprenant au moins 5 % en moles d'alcanes en C₂₊; le réacteur de CPO comprenant un catalyseur de CPO; le réacteur de CPO étant **caractérisé par** une pression de CPO, le gaz de synthèse à faible teneur en hydrogène comprenant de l'hydrogène, du monoxyde de carbone, du dioxyde de carbone, et des hydrocarbures n'ayant pas réagi; et le gaz de synthèse à faible teneur en hydrogène étant **caractérisé par** un rapport molaire d'hydrogène à monoxyde de carbone (H₂/CO) valant de 0,8 à 1,3;
(b) en option comprimer au moins une partie du gaz de synthèse à faible teneur en hydrogène pour produire un gaz de synthèse comprimé;
(c) introduire au moins une partie du gaz de synthèse et/ou du gaz de synthèse comprimé, à faible teneur en hydrogène, et de l'éther diméthylique (DME) dans une unité de carbonylation de DME pour produire de l'acétate de méthyle et un gaz de synthèse enrichi en hydrogène; l'unité de carbonylation de DME étant **caractérisée par** une pression de carbonylation de DME ; la pression du gaz de synthèse et/ou du gaz de synthèse comprimé, à faible teneur en hydrogène, étant approximativement identique à la pression de carbonylation de DME ; le gaz de synthèse enrichi en hydrogène comprenant de l'hydrogène, du monoxyde de carbone, du dioxyde de carbone, et des hydrocarbures n'ayant pas réagi, et le gaz de synthèse enrichi en hydrogène étant **caractérisé par** un rapport molaire H₂/CO valant de 1,4 à 2,2;
(d) introduire au moins une partie de l'acétate de méthyle et de l'eau dans une zone de réaction d'hydrolyse d'acétate de méthyle pour produire de l'acide acétique et un premier courant de méthanol;
(e) introduire dans une unité de synthèse de méthanol au moins une partie du gaz de synthèse enrichi en hydrogène, pour produire un second courant de méthanol et un courant de gaz de purge, le courant de gaz de purge comprenant de l'hydrogène, du monoxyde de carbone, du dioxyde de carbone, et des hydrocarbures n'ayant pas réagi;
(f) introduire au moins une partie du premier courant de méthanol et/ou au moins une partie du second courant de méthanol dans une zone de réaction de synthèse de DME pour produire un courant de DME, un réacteur commun comprenant à la fois la zone de réaction d'hydrolyse d'acétate de méthyle et la zone de réaction de synthèse de DME; et
(g) introduire au moins une partie du courant de DME dans l'unité de carbonylation de DME dans l'étape (c).

12. Procédé selon la revendication 11, dans lequel le réacteur de CPO est **caractérisé par** au moins un paramètre opérationnel de CPO choisi dans l'ensemble constitué par une température du mélange de partenaires réactionnels de CPO valant de 100 °C à 500 °C; une pression manométrique de CPO valant de 25 bar à 80 bar ; un temps de contact de CPO valant de 0,001 milliseconde (ms) à 5 secondes (s) ; un rapport molaire de carbone à oxygène (C/O) dans le mélange de partenaires réactionnels de CPO valant de 0,5:1 à 2:1, le rapport molaire C/O désignant le nombre total de moles de carbone (C) dans les hydrocarbures dans le mélange de partenaires réactionnels divisé par le nombre total de moles d'oxygène (O₂) dans le mélange de partenaires réactionnels; un rapport molaire de vapeur d'eau à carbone (V/C) dans le mélange de partenaires réactionnels de CPO inférieur à 0,25:1, le rapport molaire V/C désignant le nombre total de moles d'eau (H₂O) dans le mélange de partenaires réactionnels divisé par le nombre total de moles de carbone (C) dans les hydrocarbures dans le mélange de partenaires réactionnels; un rapport molaire de CO₂ à carbone (CO₂/C) dans le mélange de partenaires réactionnels de CPO égal ou supérieur à 0,5:1, le rapport molaire CO₂/C désignant le nombre total de moles de CO₂ dans le mélange de partenaires réactionnels divisé par le nombre total de moles de carbone (C) dans les hydrocarbures dans le mélange de partenaires réactionnels; et des associations de tels paramètres.

13. Procédé de production d'éther diméthylique (DME) comprenant les étapes consistant à:
(a) faire réagir, par une réaction d'oxydation partielle catalytique (CPO), un mélange de partenaires réactionnels de CPO dans un réacteur de CPO pour produire un gaz de synthèse à faible teneur en hydrogène; le mélange de partenaires réactionnels de CPO comprenant des hydrocarbures, de l'oxygène, du dioxyde de carbone et en option de la vapeur d'eau; les hydrocarbures comprenant au moins 3 % en moles d'alcanes en C₂₊; le réacteur de CPO comprenant un catalyseur de CPO; le réacteur de CPO étant **caractérisé par** une pression de CPO; le gaz de synthèse à faible teneur en hydrogène comprenant de l'hydrogène, du monoxyde de carbone, du dioxyde de carbone, et des hydrocarbures n'ayant pas réagi; et le gaz de synthèse à faible teneur en hydrogène étant **caractérisé par** un rapport molaire d'hydrogène à monoxyde de carbone (H₂/CO) valant de 0,8 à 1,3;
(b) introduire au moins une partie du gaz de synthèse à faible teneur en hydrogène dans un réacteur d'éther diméthylique (DME) pour produire un effluent de réacteur de DME; le réacteur de DME étant **caractérisé par** une pression de réacteur de DME; la pression de CPO et la pression de réacteur de DME étant identiques ou différentes; l'effluent de réacteur de DME comprenant du DME, du méthanol, de l'eau et du dioxyde de carbone;
(c) fractionner au moins une partie de l'effluent de réacteur de DME en un courant de DME, un courant de méthanol, un courant d'eau et un courant de dioxyde de carbone;
(d) en option recycler dans le réacteur de DME au moins une partie du courant de méthanol, et
(e) en option recycler dans le réacteur de CPO au moins une partie du courant de dioxyde de carbone.

14. Procédé selon la revendication 13, dans lequel les hydrocarbures comprennent le méthane, le gaz naturel, les liquides de gaz naturel, le gaz de pétrole liquéfié (GPL), le gaz associé, le gaz de tête de puits, le gaz enrichi, les paraffines, le gaz de schiste, les liquides de schiste, le gaz résiduel de craquage catalytique fluide (FCC), les gaz de processus de raffinerie, les gaz résiduels de raffinerie, les gaz de combustion, le gaz combustible provenant d'un collecteur de gaz combustible, ou des associations de de ceux-ci, et dans lequel les alcanes en C₂₊ comprenant l'éthane, le propane, les butanes, ou des associations de ceux-ci.

15. Procédé selon l'une quelconque des revendications 13 et **14,** dans lequel le réacteur de CPO est **caractérisé par** au moins un paramètre opérationnel de CPO choisi dans l'ensemble constitué par une température du mélange de partenaires réactionnels de CPO valant de 100 °C à 500 °C; une pression manométrique de CPO valant de 20 bar à 80 bar; un temps de contact de CPO valant de 0,001 milliseconde (ms) à 5 secondes (s); un rapport molaire de carbone à oxygène (C/O) dans le mélange de partenaires réactionnels de CPO valant de 0,5:1 à 3:1, le rapport molaire C/O désignant le nombre total de moles de carbone (C) dans les hydrocarbures dans le mélange de partenaires réactionnels divisé par le nombre total de moles d'oxygène (O₂) dans le mélange de partenaires réactionnels; un rapport molaire de vapeur d'eau à carbone (V/C) dans le mélange de partenaires réactionnels de CPO inférieur à 0,6:1, le rapport molaire V/C désignant le nombre total de moles d'eau (H₂O) dans le mélange de partenaires réactionnels divisé par le nombre total de moles de carbone (C) dans les hydrocarbures dans le mélange de partenaires réactionnels; un rapport molaire de CO₂ à carbone (CO₂/C) dans le mélange de partenaires réactionnels de CPO égal ou supérieur à 0,5:1, le rapport molaire CO₂/C désignant le nombre total de moles de CO₂ dans le mélange de partenaires réactionnels divisé par le nombre total de moles de carbone (C) dans les hydrocarbures dans le mélange de partenaires réactionnels; et des associations de tels paramètres.

16. Procédé selon l'une quelconque des revendications 13-15, excluant une étape consistant à introduire au moins une partie du gaz de synthèse à faible teneur en hydrogène dans une unité de récupération d'hydrogène pour diminuer la quantité d'hydrogène dans le gaz de synthèse à faible teneur en hydrogène.

17. Procédé selon l'une quelconque des revendications 13-16, comprenant en outre: (1) en option comprimer au moins une partie du gaz de synthèse à faible teneur en hydrogène pour produire un gaz de synthèse comprimé, la pression du gaz de synthèse comprimé étant approximativement identique à la pression de réacteur de DME; et (2) introduire dans le réacteur de DME dans l'étape (b) au moins une partie du gaz de synthèse comprimé.
